# EUROPÄISCHE PATENTSCHRIFT

(11) **EP 1 749 089 B1**
(45) Veröffentlichungstag und Bekanntmachung des Hinweises auf die Patenterteilung: **24.03.2010**
(21) Anmeldenummer: 05746415.8
(22) Anmeldetag: 09.05.2005
(51) Int. Cl.: C12N 5/00

(54) **VERFAHREN UND VORRICHTUNGEN ZUM KULTIVIEREN VON STAMMZELLEN**
METHODS AND DEVICES FOR CULTURING STEM CELLS
PROCEDES ET DISPOSITIFS POUR METTRE DES CELLULES SOUCHES EN CULTURE

(30) Priorität: 21.05.2004 DE 102004025086
(43) Veröffentlichungstag der Anmeldung: 07.02.2007
(73) Patentinhaber: Fraunhofer-Gesellschaft zur Förderung der angewandten Forschung e.V., 80686 München (DE)
(72) Erfinder: KRUSE, Charli, 23923 Herrnburg (DE); FUHR, Günter, 13187 Berlin (DE)
(74) Vertreter: Katzameyer, Michael
(86) Internationale Anmeldenummer: PCT/EP2005/004998
(87) Internationale Veröffentlichungsnummer: WO 2005/113750

(56) Entgegenhaltungen:
- EP-A- 1 304 030
- WO-A-95/17500
- WO-A-2005/068610
- DE-A1- 4 109 973

## Beschreibung

### Gegenstand der Erfindung

Die vorliegende Erfindung betrifft Verfahren zum Kultivieren von biologischen Zellen, insbesondere von Stammzellen, und Zellkulturen, die Stammzellen enthalten.

### Hintergrund der Erfindung

In der Biologie und Medizin sind zahlreiche Verfahren zum Kultivieren von biologischen Zellen allgemein bekannt. Das Kultivieren umfasst eine vorbestimmte Vermehrung der Zellen unter bestimmten Kultivierungsbedingungen. Die Vermehrung umfasst das Wachstum von Zellmaterial, wobei ggf. eine Differenzierung von Zellen erfolgt. Das Kultivieren schließt allgemein auch die Beeinflussung des Zellmaterials zur Bildung von differenzierten Zellen, Zellaggregaten, Geweben, Organen oder Organismen ein.

Es ist bekannt, die Kultivierungsbedingungen in Oberflächenkulturen einzustellen, bei denen die Zellen auf einem Substrat angeordnet und mit einem Kultivierungsmedium bedeckt sind. Oberflächenkulturen besitzen zwar Vorteile in Bezug auf die Möglichkeit der Beobachtung und Entnahme von Zellen. Nachteilig ist jedoch, dass in der Natur ein Zellwachstum auf festen Oberflächen in der Regel nicht auftritt, so dass die Kultivierungsbedingungen der Oberflächenkultur nicht den natürlichen Wachstumsbedingungen von Zellen entsprechen.

Es ist aus der Kultivierungstechnik auch bekannt, Zellen im nicht-adhärenten Zustand zum Beispiel in Schüttelkulturen oder in hängenden Tropfen zu kultivieren. Diese Techniken besitzen jedoch Beschränkungen in Bezug auf die Einstellung der Kultivierungsbedingungen und die erreichbaren Dimensionen des kultivierten Zellmaterials.

Zur Simulation natürlicher Wachstumsbedingungen wird in DE 101 09 641 A1 ein Inkubator zur Aufnahme von Nistgewebe beschrieben, der als synthetischer Uterus verwendet werden soll. Die Anwendung dieses Inkubators ist sowohl aus ethischer Sicht problematisch als auch wegen des komplexen technischen Aufbaus für anderweitige Kultivierungsaufgaben ungeeignet.

Es ist bekannt, Vogeleier zur Vermehrung von Viren zu verwenden. Diese Anwendung ist jedoch auf eine zahlenmäßige Vermehrung der Viren beschränkt.

### Aufgabe der Erfindung

Die Aufgabe der Erfindung ist es, verbesserte Verfahren zum Kultivieren biologischer Zellen, insbesondere von Stammzellen bereitzustellen, mit denen die Nachteile der herkömmlichen Techniken überwunden werden und die insbesondere eine Kultivierung von Zellen unter Kultivierungsbedingungen ermöglichen, die möglichst gut an natürliche Wachstumsbedingungen angepasst sind. Die Aufgabe der Erfindung ist es auch, eine neuartige Kultur bereitzustellen, in der die zu kultivierenden Zellen unter möglichst naturnahen Bedingungen sich vermehren und ggf. differenzieren.

Diese Aufgaben werden mit Kultivierungsverfahren und Zellkulturen mit den Merkmalen der Patentansprüche 1 und 28 gelöst. Vorteilhafte Ausführungsformen und Anwendungen der Erfindung ergeben sich aus den abhängigen Ansprüchen.

### Beschreibung der Erfindung

Verfahrensbezogen basiert die Erfindung auf der Erkenntnis der Erfinder, dass die Kultivierungsbedingungen für Stammzellen insbesondere in Bezug auf die geometrischen Bedingungen und die Nährstoffversorgung den natürlichen Wachstumsbedingungen am nächsten kommen, wenn die Kultivierung in einem Ei erfolgt. Das Ei umfasst allgemein eine Eizelle und mindestens eine Eihülle. Durch diese Komponenten wird ein natürliches Nährstoffreservoir gebildet. Erfindungsgemäß wird zum Kultivieren ein Ei von einem Wirts-Organismus (im Folgenden: Wirts-Ei) verwendet, der sich vom Donor-Organismus unterscheidet, von dem das in das Wirts-Ei eingeführte Zellmaterial stammt. Es erfolgt kein ausschließlicher Kerntransfer in das Wirts-Ei. Überraschenderweise kann das Wirts-Ei für fremde Stammzellen oder daraus gebildete Materialien als Kultivierungssubstrat verwendet werden, in dem ein Wachstum und ein Differenzieren der mindestens einen Stammzelle erfolgt.

Ein wesentlicher Vorteil der Erfindung ist es, dass eine Stammzellenkultivierung im Wirts-Ei eine vorbestimmte Differenzierung und ein geordnetes Wachstum von Geweben, Organen oder Organismen ermöglicht. Es können insbesondere Embryonen für die Fischzucht oder Tierhaltung erzeugt und aufgezogen werden oder Organgewebe von höheren Lebewesen differenziert werden.

Da das Wirts-Ei ein eigenes Nährstoffreservoir enthalten muss, werden gemäß einer bevorzugten Ausführungsform der Erfindung Eier von Wirts-Organismen als Kultivierungssubstrat verwendet, die nicht der Klasse der Säugetiere angehören. Bei Säugetieren erfolgt die Nährstoffversorgung während der Embryonalentwicklung direkt von der Mutter, so dass Säuger-Eier kein für eine längere Embryonalentwicklung ausreichendes, eigenes Nährstoffreservoir enthalten. Im Übrigen kann der Wirts-Organismus in Abhängigkeit von der konkreten Kultivierungsaufgabe und dem Donor-Organismus gewählt werden. Vorteilhafterweise können erfindungsgemäß Wirts-Eier aus den taxonomischen Klassen Vögel, Fische, Amphibien, Reptilien und Insekten gewählt werden, deren Eier sämtlich über ein ausreichendes Nährstoffreservoir verfügen. Besonders bevorzugt gehören die Donor- und Wirts-Organismen verschiedenen taxonomischen Einheiten, insbesondere Klassen, Familien oder Arten an, um die Beeinflussung der zu kultivierenden Stammzellen durch Fremdzellen möglichst gering zu halten. Bevorzugte Anwendungen der Erfindung bestehen in der Einführung von Zellmaterial, das von Säuger-Organismen stammt, in Vogel- oder Reptilieneier, oder in der Einführung von Zellmaterial, das von Fischen einer ersten Art stammt, in Fischeier einer anderen Art.

Wenn gemäß einer weiteren Ausführungsform der Erfindung zum Kultivieren des Zellmaterials ein unbefruchtetes Wirts-Ei verwendet wird, können sich in diesem vorteilhafterweise ausschließlich die fremden Zellen vermehren. Alternativ kann das fremde Zellmaterial in ein bereits befruchtetes Wirts-Ei eingeführt werden, wobei sich dann Vorteile für die Entwicklung eines optimalen Nährstoffreservoirs im Wirts-Ei ergeben können. Bei dieser Ausführungsform der Erfindung wird die im Wirts-Ei sich ggf. entwickelnde Keimscheibe entfernt oder deaktiviert, um das weitere Wachstum des fremden Zellmaterials nicht zu stören.

Gemäß einer weiteren bevorzugten Ausführungsform der Erfindung umfasst das in das Wirts-Ei eingeführte und dort kultivierte Zellmaterial eine oder mehrere adulte Stammzellen, insbesondere adulte, multipotente oder pluripotente Stammzellen, die aus einer exokrinen Drüse des Donor-Organismus stammen.

Es wurde festgestellt, dass aus exokrinen Drüsen adulter Organismen überraschenderweise pluripotente Stammzellen mit einer hohen Wachstums- und Differenzierungsfähigkeit gewonnen werden können, die die Kultivierung im Wirts-Ei eines fremden Organismus aufgrund ihrer starken und stabilen Wachstumsfähigkeit besonders gut tolerieren. Die mindestens eine im Wirts-Ei kultivierte Stammzelle stammt vorzugsweise aus exokrinem Drüsengewebe von einem Wirbeltier, z.B. einem Fisch, Amphibium, Reptil, Vogel oder Säuger, besonders bevorzugt von einem Primaten, insbesondere einem Menschen.

Das zur Gewinnung von Stammzellen für die erfindungsgemäße Kultivierung verwendete exokrine Drüsengewebe kann von einem erwachsenen Organismus, juvenilen Organismus oder nicht-humanen fötalen Organismus, vorzugsweise einem postnatalen Organismus, stammen. Der Begriff "adult", wie in der vorliegenden Anmeldung verwendet, bezieht sich somit auf das Entwicklungsstadium des Ausgangsgewebes und nicht auf dasjenige des Donororganismus, aus dem das Gewebe stammt. "Adulte" Stammzellen sind nicht-embryonale Stammzellen.

Vorzugsweise wird das erfindungsgemäß kultivierte Zellmaterial aus exokrinem Drüsengewebe aus einer Speicheldrüse, Tränendrüse, Talgdrüse, Schweißdrüse, aus Drüsen des Genitaltrakts, einschließlich Prostata, oder aus gastro-intestinalem Gewebe, einschließlich Pankreas, oder sekretorischem Gewebe der Leber isoliert. In einer stark bevorzugten Ausführungsform handelt es sich dabei um acinäres Gewebe. Ganz besonders bevorzugt stammt das acinäre Gewebe aus dem Pankreas, der Ohrspeicheldrüse oder Unterkieferspeicheldrüse.

Vorteilhafterweise sind die aus diesen Donor-Geweben bereitgestellten Stammzellen leicht zu isolieren und in einer stabilen Langzeitkultur ohne Feederzellschicht oder spezielle Zusätze im undifferenzierten Zustand zu halten. Der Begriff Feederzellen, wie in der vorliegenden Anmeldung verwendet, umfasst alle Zellen, die das Wachstum der eigentlich zu kultivierenden Zellen dadurch fördern, dass sie Wachstumsfaktoren freisetzen und/oder eine extrazelluläre Matrix bereitstellen, bzw. die Differenzierung der Stammzellkultur verhindern.

In Stammzellkulturen der Erfinder haben die Zellen nach bisher mehr als 25 Passagen über ein Jahr lang ihre Fähigkeit zur Selbsterneuerung und uneingeschränkten Teilung behalten und die Kulturen sind weiterhin stabil. Diese Eigenschaft der adulten, pluripotenten Stammzellen aus exokrinem Drüsengewebe ist besonders vorteilhaft für die Einstellung definierter Startbedingungen beim erfindungsgemäßen Kultivierungsverfahren.

Die erfindungsgemäß verwendeten adulten Stammzellen können ohne Zusatz spezieller Wachstums- oder Differenzierungsfaktoren auf einfache Weise zur Differenzierung angeregt werden, indem sie im Wirts-Ei unter räumlichen Bedingungen kultiviert werden, welche für einen dreidimensionalen Kontakt der Zellen sorgen.

Gemäß einer abgewandelten Ausführungsform der Erfindung wird im Wirts-Ei Zellmaterial kultiviert, das mindestens eine nicht-humane, embryonale Stammzelle enthält. Nicht-humane, embryonale Stammzellen sind vorteilhafterweise pluripotent und damit beim Kultivieren für Differenzierungen in alle Keimblätter geeignet.

Das erfindungsgemäß in das Wirts-Ei injizierte Zellmaterial kann gemäß einer weiteren Modifikation eine Vielzahl von Stammzellen umfassen, die ein zusammenhängendes Aggregat (oder: Zell- oder Gewebekörper) bilden. In diesem Fall können vorteilhafterweise die Chancen verbessert werden, dass die Vitalität des Materials während des Kultivierens erhalten bleibt. Des Weiteren können die Zell- oder Gewebekörper vordifferenziert sein, so dass der erfindungsgemäßen Kultivierung im Wirts-Ei eine bestimmte Differenzierungsrichtung aufgeprägt werden kann.

Die Bildung der organoiden Körper erfolgt bei einer Vorkultivierung unter Kultivierungsbedingungen, bei denen ebenfalls ein dreidimensionaler Kontakt der Stammzellen gegeben ist. In einer bevorzugten Ausführungsform erfolgt die Vorkultivierung in hängenden Tropfen, wie sie für embryonale Stammzellen bereits beschrieben wurde (Wobus et al., Biomed. Biochim. Acta 47:965-973 (1988)). Dieses Verfahren wird nachfolgend in den Beispielen noch näher beschrieben. Es versteht sich jedoch, dass alternative Kultivierungsverfahren, die für den gewünschten dreidimensionalen Kontakt der Zellen sorgen und dem Fachmann bekannt und verfügbar sind, ebenso verwendet werden können. Beispiele für solche alternativen Verfahren sind die Kultivierung in bewegter Suspensionskultur, die Kultivierung in einem elektromagnetischen Feldkäfig oder Laser-Tweezer, die Aussaat von nicht resuspendierten Primärzellen auf Kulturböden oder die Kultivierung auf Oberflächen, an denen die Zellen nicht oder schlecht haften. Solche Oberflächen können z.B. Glas, Polystyrol oder mit einer Anti-Adhäsionsschicht behandelte Oberflächen, z.B. PTFE- oder Poly-HEMA-beschichtete Oberflächen, sein.

Bei der Vorkultivierung entwickeln sich aus adulten, pluripotenten Stammzellen spontan dreidimensionale Zellverbände oder Zellaggregate, welche auch als "Organoid Bodies" oder organoide Körper bezeichnet werden. Diese organoiden Körper können in Suspensionskulturen oder Adhäsionskulturen überführt und weiter kultiviert werden. Bei ausreichender Nährstoffversorgung wachsen die organoiden Körper weiter und können Durchmesser von einigen Millimetern oder mehr erreichen. Die großen organoiden Körper zeigen eine gewebeartige Struktur und werden in diesem Stadium zur Unterscheidung von den einfachen Zellaggregaten auch als "Gewebekörper" bezeichnet.

Bringt man die organoiden Körper wieder in Oberflächenkultur, entsteht aus auswachsenden einzelnen Zellen eine zelluläre Monoschicht, aus der Multilayerbereiche hervorgehen, aus denen spontan sekundäre organoide Körper mit vergleichbaren Eigenschaften wie denjenigen der primären organoiden Körper gebildet werden. Die erfindungsgemäß verwendbaren organoiden Körper können z.B. bei der Temperatur des flüssigen Stickstoffs eingefroren gelagert werden, ohne ihre Lebensfähigkeit, Vermehrungsfähigkeit, Wachstumsfähigkeit und Differenzierbarkeit zu verlieren.

Die Bildung von embryoiden Körper aus nicht-humanen, embryonalen Stammzellen erfolgt bei der Vorkultivierung unter analogen Kultivierungsbedingungen, insbesondere wie sie für embryonale Stammzellen bereits beschrieben wurden (siehe oben, Wobus et al.).

Die Einführung oder Injektion des Zellmaterials in das Wirts-Ei kann mit an sich bekannten und für diesen Zweck geeignete Verfahren, wie z. B. eine Injektion suspendierten Zellmaterials mit einer Kapillare oder Spritze erfolgen.

Gemäß einer bevorzugten Ausführungsform der Erfindung erfolgt im Wirts-Ei ein differenziertes Wachstum des Zellmaterials. In Abhängigkeit von den geometrischen und ggf. von außen beeinflussten stofflichen Bedingungen im Wirts-Ei differenzieren sich verschiedene Stammzellen in verschiedene Richtungen. Zellen, welche aus den ursprünglich injizierten Stammzellen, organoiden Körpern oder Gewebekörpern auswachsen, können in die verschiedenen Zelltypen aller drei Keimblätter differenzieren. Zur Differenzierung sind keine Differenzierungsfaktoren notwendig und die Zellen müssen auch nicht transplantiert werden, um zu differenzieren. Es kann jedoch von Vorteil sein, solche Differenzierungsfaktoren einzusetzen, um gezielt größere Mengen eines bestimmten Zelltyps herzustellen. Solche Differenzierungsfaktoren sind im Stand der Technik bekannt und umfassen z.B. bFGF ("basic fibroblast growth factor") zur verstärkten Bildung von Herzzellen und Fibroblasten, VEGF ("vaskular endothelial growth factor"), DMSO und Isoproterenol, Fibroblastenwachstumsfaktor 4 (FGF4), Hepatozyten-Wachstumsfaktor (HGF) zur verstärkten Bildung von Herz- und Leberzellen, TGF-betal ("transforming growth factor beta1") zur verstärkten Bildung von Herzzellen, EGF ("epidermal growth factor") zur verstärkten Bildung von Haut- und Herzzellen, KGF ("keratinocyte growth factor") (machmal zusammen mit Cortison) zur Bildung von Keratinozyten, Retinsäure zur verstärkten Bildung von Nerven-, Herz- und Nierenzellen, beta-NGF ("beta nerve growth factor") zur verstärkten Bildung von Gehirn-, Leber-, Pankreas- und Nierenzellen, BMP-4 ("bone morphogenic protein 4") und Activin-A zur Bildung mesodermaler Zellen generell, sind jedoch nicht darauf beschränkt.

Differenzierte Zellen, die aus den erfindungsgemäßen verwendeten Stammzelle erhältlich sind, umfassen Knochenzellen (Osteoblasten und Osteoclasten), Chondrozyten, Adipozyten, Fibroblasten (z.B. Haut- und Sehnenfibroblasten), Muskelzellen, Endothelzellen, Epithelzellen, hematopoetische Zellen, sensorische Zellen, endokrine und exokrine Drüsenzellen, Gliazellen, neuronale Zellen, Oligodendrozyten, Blutzellen, Darmzellen, Herz-, Lungen-, Leber-, Nieren- oder Pankreaszellen, sind jedoch nicht darauf beschränkt.

Gemäß einer besonders bevorzugten Ausführungsform der Erfindung kann das differenzierte Wachstum von außen durch die Einstellung stofflicher, mechanischer und/oder thermischer Wachstumsbedingungen beeinflusst werden. Beispielsweise kann eine Temperierung des Wirts-Ei vorgesehen sein, um ein Kultivieren entsprechend dem Bebrüten eines Vogeleis zu erreichen. Mechanische Wachstumsbedingungen umfassen bspw. eine Bewegung des Wirts-Eis oder eine vorbestimmte geometrischen Ausrichtung im Gravitationsfeld. Die Einstellung stofflicher Wachstumsbedingungen umfasst vorzugsweise die Injektion von mindestens einem Differenzierungs- und/oder Wachstumsfaktor in das Wirts-Ei.

Wenn während der Kultivierung des Zellmaterials mindestens eine Eigenschaft von diesem gemessen wird, können sich besondere Vorteile für die Überwachung und Beeinflussung des Wachstumsverlaufs ergeben. Vorzugsweise wird mit der Messung erfasst, ob das kultivierte Zellmaterial noch lebt. Die Vitalitätsmessung umfasst bspw. eine NMR- oder Ultraschallmessung. Alternativ oder zusätzlich kann eine Erfassung eines Differenzierungsgrades des Zellmaterials vorgesehen sein. Vorteilhafterweise können in Abhängigkeit vom Ergebnis der Messung die Kultivierungsbedingungen verändert werden.

Gemäß einer weiteren Variante ist erfindungsgemäß vorgesehen, dass die Kultivierung des Zellmaterials im Wirts-Ei abgebrochen wird, wenn ein vorbestimmter Kultivierungszustand erreicht wurde. Durch diesen Abbruch können ggf. fehldifferenzierte oder unerwünscht wuchernde Zellmaterialien von einem weiteren Wachstum gehindert werden. Die zum Abbruch der Kultivierung herangezogenen Kultivierungsbedingungen umfassen vorzugsweise die Kultivierungszeit, die erreichte Größe des kultivierten Materials und/oder den erreichten Differenzierungsgrad des kultivierten Zellmaterials.

Gemäß einer weiteren Abwandlung kann vorgesehen sein, dass kultiviertes Zellmaterial aus dem Ei entnommen wird, ohne, dass die Eihülle beschädigt wird. Es kann beispielsweise laufend differenziertes Zellmaterial aus dem Ei abgezogen werden.

Erfindungsgemäß kann das stammzellbasierte Zellmaterial an einer oder mehreren Positionen im Wirts-Ei angeordnet werden. Bevorzugt ist die Injektion des Zellmaterial an den Ort im Wirts-Ei, der der natürlichen Position der Keimscheibe im unbeeinflussten Ei entspricht. Diese Ausführungsform der Erfindung ist besonders vorteilhaft, da die Kultivierung unter geometrischen und stofflichen Bedingungen beginnt, die den natürlichen Brutbedingungen am nächsten kommen.

Vorzugsweise wird das Zellmaterial in einer Kultivierungsblase im Wirts-Ei positioniert, die mindestens eine flüssige oder gelförmige Kultivierungssubstanz enthält. Dies ermöglicht die gezielte Vorgabe von stofflichen Kultivierungsbedingungen im Wirts-Ei. Gemäß bevorzugten Varianten der Erfindung kann die Kultivierungssubstanz in der Kultivierungsblase laufend oder nach vorgebbaren Kultivierungsprotokollen in bestimmten Zeitabständen, z. B. periodisch ausgetauscht werden.

Die Kultivierungssubstanz kann auch in der Umgebung des Zellmaterials am Ort des Nährstoffreservoirs des Wirts-Ei angeordnet werden, indem das Nährstoffreservoir (insbesondere Eidotter, Eiweiß) verdrängt oder zumindest teilweise ausgetauscht wird. Der Vorteil dieser Ausführungsform der Erfindung besteht darin, dass zwar die geometrischen Kultivierungsbedingungen im Wirts-Ei unverändert bleiben, die stofflichen Kultivierungsbedingungen jedoch an die Anforderungen des Donor-Organismus angepasst werden können. Auch im Nährstoffreservoir kann die Kultivierungssubstanz während der Kultivierung des Zellmaterials laufend oder in vorgebbaren Zeitabständen erneuert oder ausgetauscht werden.

Erfindungsgemäß kann vorgegeben sein, dass das Eiweiß des Wirts-Ei komplett durch die Kultivierungssubstanz ausgetauscht wird. Des Weiteren kann ein kompletter Austausch des Eidotter vorgesehen sein, wobei jedoch die geometrische Anordnung beider Komponenten und die Eidotterumhüllung erhalten bleiben.

Vorzugsweise umfasst der Austausch ein Einbringen von Tropfen der Kultivierungssubstanz in das Nährstoffreservoir des Wirts-Ei. Die flüssigen oder gelförmigen Tropfen können eine Membran-Umhüllung aufweisen, durch die Wirkstoffe, zum Beispiel Hormone oder Differenzierungsfaktoren in das Ei diffundieren können. Es ist zum Beispiel eine Lipid-Membran vorgesehen.

Wenn gemäß einer weiteren Ausführungsform der Erfindung während der Kultivierung des Zellmaterials im Wirts-Ei bestimmte Stoff- und/oder Temperaturgradienten erzeugt werden, kann die Kultivierung des Zellmaterials vorteilhafterweise nach vorgebbaren stofflichen und/oder geometrischen Kultivierungsprotokollen erfolgen. Ein besonderer Vorteil von natürlichen Wirts-Eiern besteht darin, dass durch die Eihülle selbst bei der Bildung einer Kalkschale sowohl ein stofflicher als auch ein thermischer Einfluss auf das Innere des Zellmaterials ausgeübt werden kann.

Erfindungsgemäß ist es auch möglich, das Zellmaterial im Wirts-Ei in Anwesenheit von mindestens einem Feststoff zu kultivieren. Mit dem Feststoff können vorteilhafterweise geometrische Begrenzungen für die Stammzellenkultivierung und -differenzierung vorgegeben werden. Wenn als Feststoff bspw. mindestens ein Trägerpartikel verwendet wird, kann dieser ein Substrat bilden, das während der Kultivierung umwachsen wird. Diese Anwendung der Erfindung ist für die Erzeugung von Implantatmaterialien von Vorteil. Des Weiteren können auch Feststoffe zunächst mit einem Abstand vom Zellmaterial angeordnet werden, um bei dessen Wachstum eine geometrische Begrenzung zu bilden. Derartige geometrische Grenzen können sogar in mmbis cm-Bereich erzeugt werden. Hierzu werden bspw. Flüssigkeiten in das Wirts-Ei im flüssigen Zustand (z. B. flüssige Polymere) eingebracht und dann lokal z. B. unter dem Einfluss von Wärme oder Licht ausgehärtet.

Gemäß einem unabhängigen Gesichtspunkt der Erfindung wird die o. g. Aufgabe stoffbezogen durch eine Zellkultur gelöst, die eine Zusammensetzung aus einem Wirts-Ei und mindestens einer Stammzelle umfasst, die in dem Wirts-Ei angeordnet ist. Der Donor-Organismus, aus dem die adulte, pluripotente oder nicht-humane, embryonale Stammzelle gewonnen wurde, ist nicht mit dem Wirts-Organismus identisch, aus dem das Wirts-Ei gewonnen wurde. Vorzugsweise gehören der Wirts-Organismus und der Donor-Organismus sogar unterschiedlichen taxonomischen Klassen an.

Zur Durchführung des erfindungsgemäßen Verfahrens geeignet ist eine Zellkultureinrichtung, die eine Inkubatoreinrichtung aufweist, die zur Aufnahme eines Ei von einem Wirts-Organismus eingerichtet ist. Hierzu besitzt die Inkubatoreinrichtung einen Inkubatorraum, der größer als das Wirts-Ei ist Vorzugsweise ist die innere Form des Inkubatorraums zumindest in Teilbereichen an die äußere Form des Wirts-Eis angepasst. Diese Zellkultureinrichtung besitzt vorteilhafterweise eine besonders einfachen Aufbau, da lediglich eine Aufnahme für das Wirts-Ei mit dem zu kultivierenden Zellmaterial bereitgestellt werden muss. Im Unterschied zu herkömmlichen Inkubatoreinrichtungen benötigt diese Zellkultureinrichtung kein gesondertes Kultivierungssubstrat, da dieses durch das in den Inkubatorraum einsetzbare Wirts-Ei gebildet wird.

Vorteile für eine breite und flexible Anwendung dieser Zellkultureinrichtung ergeben sich, wenn diese mit einer Injektionseinrichtung zur Einführung von Lösungen oder Suspensionen in das Innere des Wirts-Eis, einer Temperiereinrichtung zur Temperierung des Inkubatorraumes, einer Befeuchtungseinrichtung zur Einstellung einer vorbestimmten Feuchte im Inkubatorraum, einer Messeinrichtung zur Erfassung von Eigenschaften des Wirts-Ei oder des kultivierten Zellmaterials und/oder einer Antriebseinrichtung zur Bewegung des Wirts-Ei in der Inkubatoreinrichtung ausgestattet ist. Die Injektionseinrichtung umfasst bspw. einen Zellinjektor zur Einführung von Suspensionen aus Zellen oder Zellaggregaten, wie z. B. Zellkörpern. Der Zellinjektor besitzt vorzugsweise eine starre Injektionsleitung, z. B. eine Injektionsnadel mit der im Wirts-Ei an vorbestimmten Positionen eine Ablage von Zellmaterial erfolgen kann. Zur Einführung von Wachstums- oder Differenzierungsfaktoren weist die Injektionseinrichtung des Weiteren einen Flüssigkeitsinjektor auf. Vorzugsweise werden der Zell- und Flüssigkeitsinjektor durch eine gemeinsame Komponente gebildet.

Einen unabhängigen Gegenstand der Erfindung stellt die Verwendung eines Ei eines Wirts-Organismus zur Kultivierung von biologischen Zellen dar, die nicht vom Wirts-Organismus stammen. Besonders bevorzugt ist die Verwendung von einem Ei, insbesondere einem Vogel- oder Fischei, als Inkubator für von außen in das Ei eingesetzte Stammzellen.

Eine bevorzugte Anwendung der Erfindung besteht in der Entwicklung gewebeähnlicher oder organähnlicher multizellulärer Systeme in vitro. In einer bevorzugten Ausführungsform umfassen die multizellulären Systeme dabei verschiedene Zellarten. Diese multizellulären Systeme können dann beispielsweise transplantiert werden oder extrakorporal, z.B. zur Blutwäsche oder zur Produktion gewünschter Substanzen, oder als pharmakologisches Gewebemodell eingesetzt werden.

### Kurzbeschreibung der Figuren

Weitere Einzelheiten und Vorteile der Erfindung werden im Folgenden unter Bezug auf die beigefügten Zeichnungen beschieben.
Figur 1 zeigt ein Flussdiagramm zur Illustration von Schritten des erfindungsgemäßen Verfahrens.
Figur 2 zeigt schematisch die Übertragung von Stammzellen in ein Vogelei.
Figur 3 zeigt Einzelschritte der Präparation von Zellmaterial.
Figur 4 zeigt schematisch verschiedene Varianten der Übertragung von Zellmaterial in ein Vogelei.
Figur 5 zeigt Einzelschritte der Deposition von Zellmaterial im Wirts-Ei.
Figur 6 zeigt weitere Einzelheiten der Ablage von Zellmaterial und Zusatzstoffen in einem Vogelei.
Figur 7 zeigt Einzelschritte der Kultivierung von Zellmaterial im Wirts-Ei.
Figur 8 zeigt schematisch eine Schnittansicht einer für das erfindungsgemäße Verfahren geeigneten Kultivierungseinrichtung.
Figur 9 zeigt schematisch die Kultivierung der Stammzellen in Oberflächenkultur und in hängenden Tropfen sowie die Bildung und weitere Kultivierung von organoiden Körpern.

### Beschreibung bevorzugter Ausführungsbeispiele

Bei der folgenden Beschreibung bevorzugter Ausführungsbeispiele der Erfindung wird auf die Kultivierung adulter, pluripotenter Stammzellen in einem Wirts-Ei beispielhaft Bezug genommen. Die Erfindung ist analog bei der Kultivierung nicht-humaner, embryonaler Stammzellen anwendbar, wobei deren Gewinnung und ggf. Vorkultivierung zu embryoiden Körpern an sich bekannt sind und hier nicht beschrieben werden.

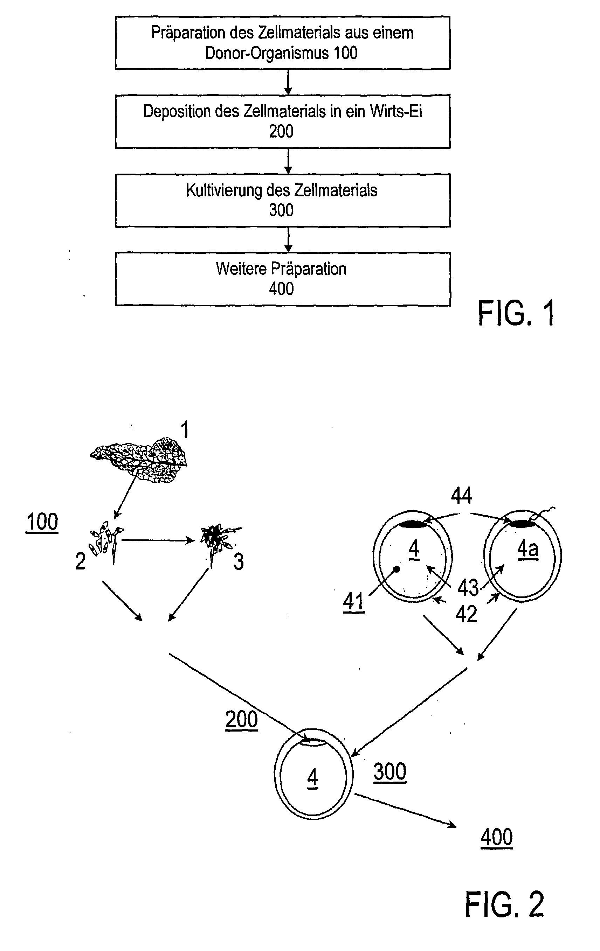

Allgemein umfasst das erfindungsgemäße Verfahren die Einführung und Kultivierung von mindestens einer Stammzelle in ein Wirts-Ei eines fremden Wirts-Organismus. Die Umsetzung dieses Verfahrens einschließlich von ggf. vorgesehenen Vorbereitungs- und Folgeschritten ist schematisch in Figur 1 illustriert, wobei weitere Einzelheiten unten erläutert sind.

Zuerst erfolgt bei Schritt 100 die Präparation des Zellmaterials, das mindestens eine Stammzelle umfasst und in das Wirts-Ei eingeführt werden soll. Die Präparation umfasst die unten mit weiteren Einzelheiten beschriebene Isolation der Stammzellen aus dem Donor-Organismus und ggf. deren Vorkultivierung zu organoiden Körpern oder Gewebekörpern. Anschließend wird bei Schritt 200 das Zellmaterial im Wirts-Ei deponiert. Unmittelbar nach der Einführung und Deposition beginnt das Wachstum und die Differenzierung der Stammzellen (Kultivierung bei Schritt 300). In Abhängigkeit vom Kultivierungsprotokoll können sich bei Schritt 400 weitere Präparations- oder Bearbeitungsprozeduren anschließen.

Weitere Einzelheiten der Schritte 100 bis 400 sind in Figur 2 illustriert. Der Präparationsschritt 100 umfasst die Isolation der Stammzellen 2 aus dem exokrinen Drüsengewebe, z. B. dem Pankreas 1 eines Säugetieres, z. B. einer Maus. Die Stammzellen 2 aus der Primärkultur können dann bei Schritt 200 in das Wirts-Ei eingeführt werden. Alternativ ist es möglich, aus den Stammzellen 2 durch eine Vorkultivierung und Aggregation zunächst die organoiden Körper oder Gewebekörper 3 zu bilden, die dann in das Wirts-Ei eingeführt werden.

Das Wirts-Ei 4 ist bspw. ein Vogel-, Reptilien- oder Fischei, das eine Eizelle 41 und mindestens eine Eihülle 42 besitzt. Die Eizelle 41 umfasst Eidotter 43 und eine Keimscheibe 44. Im rechten Teil von Figur 2 ist illustriert, dass zur erfindungsgemäßen Kultivierung des stammzellbasierten Zellmaterials alternativ unbefruchtete Eier 4 oder bereits befruchtete Eier 4a verwendet werden können.

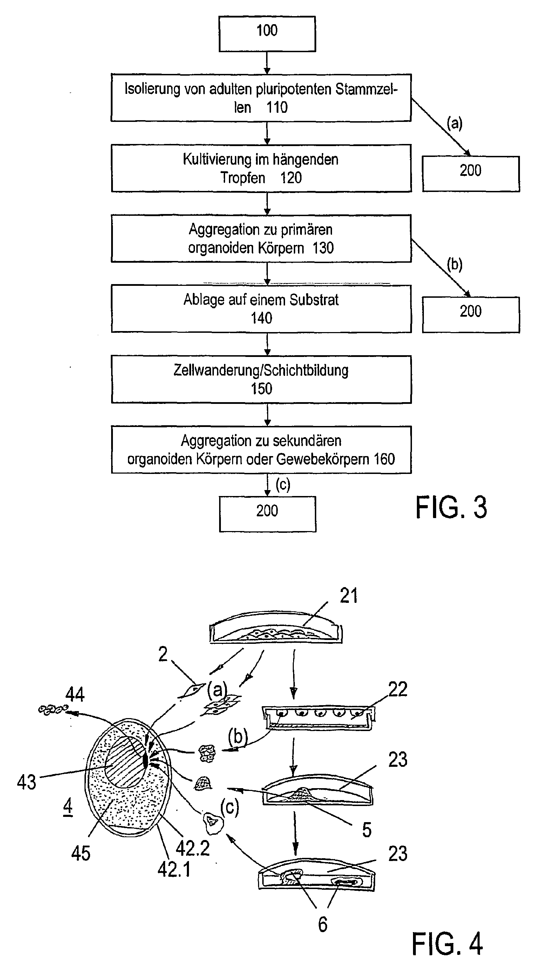

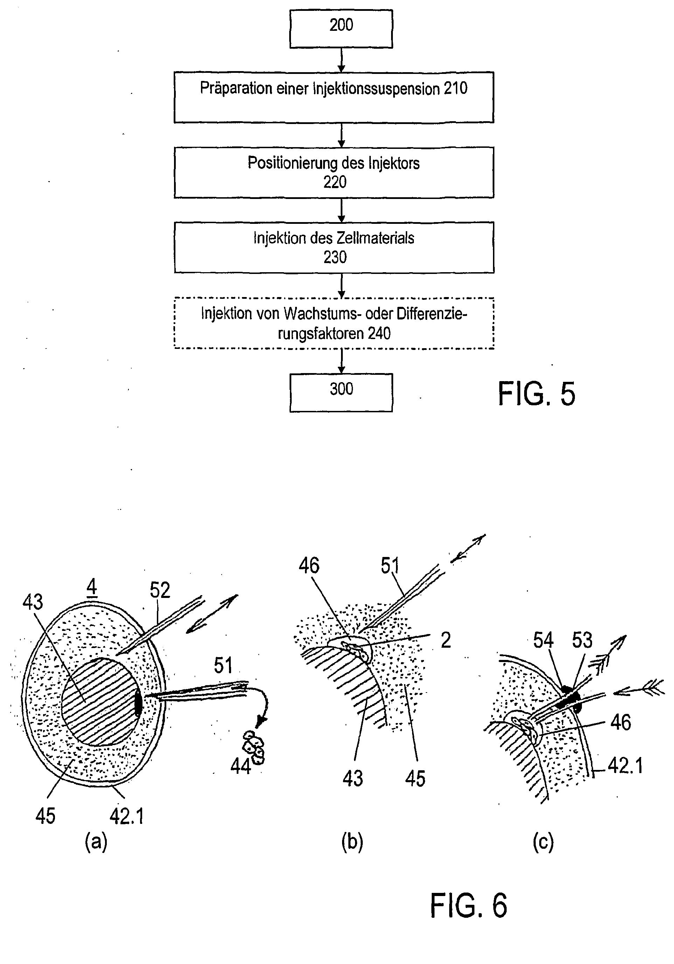

In Figur 3 sind die verschiedenen Möglichkeiten gezeigt, Stammzellen, organoide Körper oder Gewebekörper als Ausgangsmaterialien für die Kultivierung bereitzustellen. Zuerst ist bei Schritt 110 die Isolierung der adulten, pluripotenten Stammzellen als einem Donor-Organismus vorgesehen. Gemäß der ersten Variante (a) erfolgt die Deposition 200 des Zellmaterials, in dem die primär gewonnenen Stammzellen in das Wirts-Ei eingeführt werden. Alternativ erfolgt als nächstes die unten mit weiteren Einzelheiten beschriebene Kultivierung der isolierten Stammzellen im hängenden Tropfen (Schritt 120, siehe Figur 5) mit der Aggregation zu den so genannten primären organoiden Körpern (Schritt 130). Gemäß Variante (b) können die primären organoiden Körper direkt zur Kultivierung im Wirts-Ei deponiert (Schritt 200 in Figur 1) werden. Gemäß einer weiteren Alternative erfolgt als nächstes die Ablage der primären organoiden Körper auf einem Substrat (Schritt 140) zur Schaffung einer Adhäsionskultur (siehe auch Figur 4). Die Erfinder haben festgestellt, dass die in Suspensionskultur wachsenden primären organoiden Körper in Adhäsionskultur zu größeren Gewebekörpern wachsen oder neue organoide Körper bilden können. Auf dem Substrat folgt durch eine Zellwanderung die Bildung einer Monoschicht (Schritt 150) und aus dieser die Aggregation zu den so genannten sekundären organoiden Körpern (Schritt 160). Diese können gemäß Variante (c) wiederum direkt zur Kultivierung deponiert werden (Schritt 200) verwendet werden.

Die Varianten (a) bis (c) sind auch in Figur 4 illustriert. Das Wirts-Ei 4 ist z. B. ein Hühnerei mit einem Nährstoffreservoir aus Eidotter 43 und Eiweiß 45. Das Nährstoffreservoir ist von einer Kalkschale 42.1 umgeben, die zusammen mit einer Eihaut 41.2 die Eihülle bildet. Auf der Grenzfläche zwischen dem Eidotter 43 und dem Eiweiß 45 befindet sich die Keimscheibe 44. Bei einem Vorbereitungsschritt wird die Keimscheibe 44 aus dem Wirts-Ei 4 entfernt. Dies erfolgt mit mechanischen Mitteln bspw. durch Absaugen mit einer Kanüle. Alternativ kann die Keimscheibe 44 durch den Zusatz einer chemischen Substanz (z. B. mit einem aus der Mikroskopie an sich bekannten Letal-Farbstoff oder Hydroxyharnstoff) oder durch eine radioaktive Bestrahlung (z. B. mit γ-Strahlen) deaktiviert werden. Anschließend wird im Wirts-Ei 4 am Ort der natürlichen Position der Keimscheibe das stammzellbasierte Zellmaterial deponiert. Gemäß Variante (a) werden einzelne Stammzellen 2 oder eine Vielzahl von Stammzellen 2 direkt aus der Primärkultur 21 in das Wirts-Ei 4 eingeführt. Die Positionierung erfolgt vorzugsweise unterhalb oder oberhalb der Vitellinmembran des Eidotter, ohne diese Membran zu zerstören. Wenn zunächst die Hängetropfenkultur 22 gebildet wird (Schritt 120 in Fig. 3), können Zellaggregate aus der Hängetropfenkultur 22 gemäß Variante (b) im Wirts-Ei 4 deponiert werden. Die Deposition der sekundären organoiden Körper 5, die in der.Adhäsionskultur 23 gebildet wurden, ist bei Variante (c) illustriert. Wenn die primären organoiden Körper zu Gewebekörpern 6 wachsen, können diese ebenfalls aus der Adhäsionskultur 23 entnommen und in das Wirts-Ei 4 eingeführt werden. Die Gewebekörper 6 sind durch ein Wachstum von organoiden Körpern in der Adhäsionskultur entstanden und besitzen einen charakteristischen Durchmesser von einigen hundert Mikrometern bis zu einigen Millimetern.

Die Präparation und Vorkultivierung von nicht-humanen, embryonalen Stammzellen und embryoiden Körpern erfolgt analog zu den illustrierten Varianten (a) bis (c).

Weitere Einzelheiten des Depositionsschrittes 200 gemäß Figur 1 sind beispielhaft in Figur 5 zusammengestellt. Nach der Präparation des zu kultivierenden Zellmaterials wird zuerst bei Schritt 210 eine Injektionssuspension gebildet. Die Injektionssuspension umfasst das Zellmaterial, z. B. eine Vielzahl von Stammzellen oder organoiden Körpern, und eine Trägerflüssigkeit. Die Trägerflüssigkeit ist bspw. ein physiologische Nährlösung (z. B.: HEPES-Eagle-Medium, Inkubationsmedium DMEM oder dgl.). Bei Schritt 210 kann die Verbindung des Zellmaterials mit Substraten oder Trägerpartikeln, z. B. Kunststoff- oder Glaskugeln vorgesehen sein, die als Feststoff mit dem Zellmaterial in das Wirts-Ei eingeführt werden sollen. Anschließend wird bei Schritt 220 der Injektor (siehe Figur 6) zur Einführung des Zellmaterials positioniert. Der Injektor wird durch die Eihülle und das Nährstoffreservoir bis zur gewünschten Ablageposition am Ort der Keimscheibe bewegt. Beispielsweise wird eine Injektionsnadel mit einer Positioniereinrichtung relativ zum gewünschten Ablageort ausgerichtet und mit einer Antriebseinrichtung in das Wirts-Ei eingeführt. Die korrekte Positionierung des Injektors kann bspw. durch eine Ultraschall-Messung geprüft werden. Anschließend folgt bei Schritt 230 die Injektion des Zellmaterials mit dem Injektor. In Abhängigkeit von der Kultivierungsaufgabe können anschließend bei Schritt 240 weitere Wachstums- und/oder Differenzierungsfaktoren in das Wirts-Ei in die unmittelbare Umgebung des injizierten Zellmaterials eingeführt werden.

Figur 6 illustriert verschiedene Phasen der Vorbereitung und Beschickung des Wirts-Ei 4. Teilbild (a) zeigt das Ei 4 mit dem Eidotter 43, dem Eiweiß 45 und der Keimscheibe 44 innerhalb der Kalkschale 42.1. Beispielhaft sind ein Suspensionsinjektor 51 und ein Flüssigkeitsinjektor 52 gezeigt, die jeweils durch eine Kapillare oder Spritzennadel gebildet werden und in einer Richtung entlang ihrer Längsausdehnung verfahrbar sind (siehe Doppelpfeil). Mit dem Suspensionsinjektor 51 kann zuerst die Keimscheibe 44 entnommen werden. Anschließend wird der Suspensionsinjektor 51 zur oben beschriebenen Einführung des Zellmaterials benutzt. Der Flüssigkeitsinjektor 52 dient beispielsweise der Übertragung von anderen, die Kultivierung des Zellmaterials beeinflussenden Komponenten, wie z. B. von Wachstums- und/oder Differenzierungsfaktoren. Diese Komponenten können bei der Präparation des Wirts-Ei 4 vor der Kultivierung und/oder während der Kultivierung des Zellmaterials im Wirts-Ei 4 zugeführt werden.

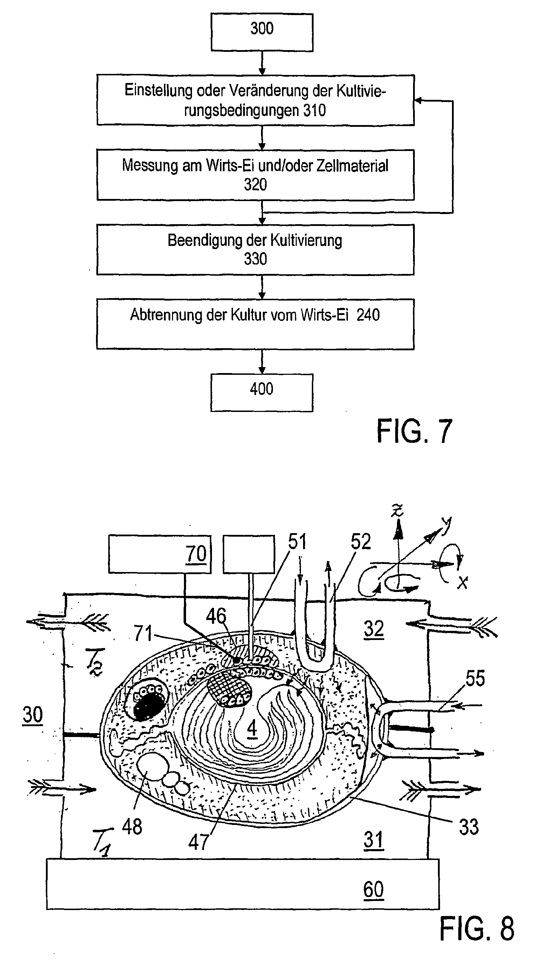

Die Entnahme und Einführung von Zellen oder chemischen Substanzen erfolgt, indem mit der Spitze des Injektors in der Kalkschale 42.1 ein Loch gebildet wird. Die Bildung des Loches und weitere Betätigung der Injektoren erfolgt unter sterilen Bedingungen. Teilbild (b) der Figur 7 zeigt den Ausschnitt im Inneren des Wirts-Ei 4, in dem mit dem Suspensionsinjektor 51 an der Grenze zwischen dem Eidotter 43 und dem Eiweiß 45 am Ort der vorher entfernten Keimscheibe die Stammzellen 2 angeordnet wurden. Die Stammzellen 2 befinden sich in einer Kultivierungsblase 46, die durch die Suspensionsflüssigkeit gebildet wird und ggf. Wachstums- und/oder Differenzierungsfaktoren enthält.

In Teilbild (c) ist die Versorgung der Kultivierungsblase 46 gezeigt. Erfindungsgemäß können während der Kultivierung von der Kultivierungsblase 46 eine oder mehrere Flüssigkeitsleitungen 53 nach außen führen (siehe Figur 8), um Kultivierungsmedium zuzuführen oder Stoffwechselprodukte abzuführen. Die Flüssigkeitsleitungen 53 werden durch eine Öffnung in der Kalkschale 42.1 geführt, die mit einer Dichtung 54 verschlossen ist. Die Dichtung 54 besteht bspw. aus Paraffin oder einem anderen plastisch verformbaren Polymer.

Über die Flüssigkeitsleitungen 53 kann durch die Kultivierungsblase 46 eine Lösungsdurchströmung bereitgestellt werden, mit der bspw. das Volumen der Kultivierungsblase entsprechend vorgebbaren Kultivierungsprozeduren verringert oder vergrößert werden kann. Des Weiteren können über die Flüssigkeitsleitungen 53 veränderte Kultivierungslösungen, z. B. mit wechselnden Zusätzen (z. B. Hormone, Differenzierungsfaktoren) eingespült oder weitere biologische Zellen, wie z. B. Stammzellen oder vordifferenzierte Zellen eingeführt werden. Entsprechend ist auch die Entnahme von kultiviertem Zellmaterial aus der Kultivierungsblase 46 möglich.

Figur 7 illustriert weitere Einzelheiten des Schrittes 300 der Kultivierung des Zellmaterials (siehe Figur 1). Nach der Ablage des Zellmaterials bei Schritt 200 erfolgt die Einstellung von Kultivierungsbedingungen im Wirts-Ei 4, falls dies nicht bereits im Rahmen einer Vorbereitung des Wirts-Ei erfolgt ist. Die Kultivierungsbedingungen werden möglichst physiologisch oder naturnah gewählt. So wird bspw. eine Kultivierungstemperatur entsprechend der typischen Körpertemperatur des Donor-Organismus gewählt. Des Weiteren kann eine Bewegung des Wirts-Ei vorgesehen sein, die der von Vögeln praktizierten Umwälzbewegung an bebrüteten Eiern entspricht.

Bei Schritt 320 ist mindestens eine Messung am Wirts-Ei 4 und/oder dem kultivierten Zellmaterial vorgesehen, um den Fortgang der Kultivierung oder das Erreichen eines bestimmten Kultivierungszustandes festzustellen. Das Messergebnis wird mit einem vorgegebenen Kultivierungsprotokoll verglichen. In Abhängigkeit vom Vergleich wird die Kultivierung ggf. beendet (Schritt 330). Alternativ wird die Kultivierung unter den gegebenen Bedingungen oder unter veränderten Kultivierungsbedingungen (Rücksprung zu Schritt 310) fortgesetzt. Schließlich erfolgt nach Beendigung der Kultivierung eine Abtrennung der kultivierten Zellen vom Wirts-Ei bei Schritt 240.

Figur 8 zeigt ein Ausführungsbeispiel einer zur Durchführung des Verfahrens erfindungsgemäßen geeigneten Zellkultureinrichtung. Die Zellkultureinrichtung umfasst eine Inkubatoreinrichtung 30 mit einem Bodenteil 31 und einem Deckelteil 32, die im zusammengesetzten Zustand einen Inkubatorraum 33 zur Aufnahme des Wirts-Ei 4 bilden. Die Boden- und Deckelteile 31, 32 besitzen mehrere Funktionen. Neben der Formung des Inkubatorraums 33 dienen sie auch der Temperierung und ggf. dem Stoffaustausch. Die Boden- und Deckelteile 31, 32 bilden eine Temperierungseinrichtung und/oder Befeuchtungseinrichtung. Hierzu sind die Boden- und Deckelteile 31, 32 jeweils hohle Bauteile, die mit Temperierungs- und/oder Versorgungsflüssigkeiten (oder entsprechenden Gasen) gefüllt oder von diesen durchströmt werden können. Zur Erzielung des Stoffaustausches besitzen die Boden- und Deckelteile 31, 32 an den zum Inkubatorraum 33 weisenden Seiten zumindest in Teilbereichen eine semipermeable Wand, durch die Substanzen in das Wirts-Ei diffundieren können.

Die Zellkultureinrichtung ist des Weiteren mit einer Antriebseinrichtung 60 ausgestattet, mit der die Inkubatoreinrichtung 20 in allen Raumrichtungen verschwenkbar ist (siehe Koordinatensystem). Die Antriebseinrichtung 60 umfasst bspw. einen aus der Labortechnik bekannten Schwenkantrieb und/oder Drehteller-Antriebe.

Die Zellkultureinrichtung enthält weitere Komponenten, die die Injektionseinrichtung 50 bilden. Teile der Injektionseinrichtung 50, die einzeln oder gemeinsam vorgesehen sein können, umfassen den Suspensionsinjektor 51, einen Flüssigkeitsinjektor 52 und einen Gasinjektor 55. Die Flüssigkeits- und Gasinjektoren 52, 55 umfassen Flüssigkeits- und Gasleitungen, die durch einen Teilbereich des Wirts-Ei 4 geführt werden und zumindest in diesem Teilbereich eine semipermeable Wand besitzen. Dadurch können durch Diffusion Substanzen mit dem Flüssigkeitsinjektor 52 bspw. in das Eiweiß oder mit dem Gasinjektor 55 in die Gasblase des Wirts-Ei 4 eingeführt werden. Das Bezugszeichen 56 verweist allgemein auf eine Positionier- und Antriebseinrichtung für den Suspensionsinjektor 51. Die Positionier- und Antriebseinrichtung enthält zum Beispiel einen piezoelektrischen Antrieb.

Das Bezugszeichen 70 verweist allgemein auf eine berührungslos oder in Kontakt mit dem Wirts-Ei arbeitende Messeinrichtung. Die Messeinrichtung 70 zum berührungslosen Betrieb ist bspw. eine NMR-Messeinrichtung oder eine Ultraschallmesseinrichtung. Für den Kontaktbetrieb ist die Messeinrichtung 70 mit einer Sonde 71 ausgestattet, mit der lokal Messungen im Wirts-Ei 4 z. B. am kultivierten Zellmaterial durchgeführt werden können. Die Sonde 71 kann bspw. für Temperaturmessungen, elektrische Messungen oder optische Messungen (z. B. Fluoreszenzmessungen) gerichtet sein.

Figur 8 zeigt illustriert die Kultivierung des Zellmaterials in einer Kultivierungsblase 46 ober- oder unterhalb der Membran 47 zwischen Eidotter und Eiweiß. Die alternativ mögliche Kultivierung ohne eine Kultivierungsblase ist ebenfalls gezeigt. Das Bezugszeichen 48 verweist auf Tropfen mit einer Kultivierungssubstanz, die von außen in das Eiweiß eingebracht wurden.

### Weitere Beispiele

In den folgenden, nicht-beschränkenden Beispielen wird die vorliegende Erfindung näher erläutert.

Die allgemeinen Arbeitsanweisungen, wie sie für Verfahren zur Kultivierung von tierischen Zellen und insbesondere von Säugetierzellen gebräuchlich sind, sind zu beachten. Eine sterile Umgebung, in der das Verfahren durchgeführt werden soll, ist - auch wenn hierzu keine weitere Beschreibung erfolgt - in jedem Fall einzuhalten. Folgende Puffer und Medien wurden verwendet:

| | |
|---|---|
| HEPES-Stammlösung (pH 7,6) | 2,3 83 g HEPES auf 100 ml A. *bidest.* |
| HEPES-Eagle-Medium (pH 7,4) | 90 ml Modified Eagle Medium (MEM) |
| | 10 ml HEPES-Stammlösung |
| Isolationsmedium (pH 7,4) | 32 ml HEPES-Eagle-Medium |
| | 8 ml 5% BSA in A. bidest. |
| | 300 µl 0,1 M CaC12 |
| | 100 µl Trasylol (200.000 KIE) |
| Digestionsmedium (pH 7,4) | 20 ml Isolationsmedium |
| | 4 ml Kollagenase (Kollagenase NB 8 von Serva) |
| Inkubationsmedium | Dulbecco's Modified Eagle Medium (DMEM) |
| Nährmedium | Dulbecco's Modified Eagle Medium (DMEM) |
| | DMEM + 4500 mg/l Glucose |
| | + L-Glutamin |
| | - Pyruvat |
| | + 20 % FKS (inaktiviert) + 1 ml/100 ml |
| | Pen/Strep |
| | (10000 E/10000 µg/ml) |
| | oder |
| | DMEM + 10 % Eigenplasma + 1 ml/100 ml |
| | Pen/Strep, |
| | vor Gebrauch auf 37°C erwärmen |
| Differenzierungsmedium | 380 ml DMEM |
| | 95 ml 30 min bei 54 °C inaktiviertes FKS |
| | 5 ml Glutamin (GIBCO BRL) |
| | 5 ml (3,5 µl β-Mercaptoethanol auf 5 ml PBS) |
| | 5 ml Non-essential amino acids (GIBCO BRL) |
| | 5 ml Penicillin/Streptomycin (GIBCO BRL) |
| | (10000 E/10000 µg/ml) |

Statt fötalem Kalbserum (FKS) im Nährmedium und Differenzierungsmedium kann gegebenenfalls auch Eigenplasma oder, weniger bevorzugt, Eigenserum des Gewebedonors verwendet werden.

Dies ist insbesondere dann von Bedeutung, wenn der Gewebedonor mit dem späteren Empfänger der Stammzellen oder davon abgeleiteten differenzierten Zellen identisch ist. Eine solche autologe Behandlung ist zur Verhinderung einer eventuellen Abstoßungsreaktion bevorzugt.

Das Nährmedium kann als Basismedium statt des verwendeten DMEM-Mediums auch ein anderes für die Kultivierung von eukaryotischen Zellen, insbesondere Säugerzellen, bekanntes, geeignetes Basismedium enthalten, in dem die differenzierten Zellen absterben und sich die gewünschten Stammzellen vermehren. Auch Isolationsmedium, Inkubationsmedium und Differenzierungsmedium können ein anderes übliches und geeignetes Basismedium enthalten.

Die folgenden Beispiele 1 und 2 beschreiben detailliert zwei Arbeitsprotokolle zur Isolierung und Kultivierung adulter pluripotenter Stammzellen aus acinärem Gewebe des Pankreas. Beispiel 3 beschreibt ein entsprechendes Protokoll für die Isolierung aus acinärem und tubulösem Gewebe der Speicheldrüse.

### BEISPIEL 1

### 1. Präparation des Gewebes und Isolierung der Zellen

In den *Ductus pancreaticus* von 2-3 Jahre alten Ratten werden mittels einer Spritze und einer stumpfen Kanüle bei der Ratte 10 ml Digestionsmedium langsam und luftblasenfrei injiziert. Das gesamte Pankreas wird dadurch aufgeblasen und kann so besser herauspräpariert werden. Das Pankreas wird dann in ein Becherglas überführt und weitere 5 ml Digestionsmedium dazugegeben. Nachdem man das Fettgewebe und Lymphknoten entfernt hat, wird das Gewebe im Becherglas mit einer feinen Schere sehr fein zerkleinert, oben schwimmendes Fettgewebe abgesaugt und die Suspension wird abschließend 1 min mit Carbogen begast (wenn nötig wiederholen) und für 20 min bei 37 °C mit Alufolie bedeckt in einem Schüttler bei 200 Zyklen/min inkubiert. Danach saugt man das Medium vorsichtig ab, zerkleinert erneut mit einer Schere das Gewebe und wäscht die Gewebestücke zweimal mit je 10 ml Isolationsmedium und gibt wieder 5 ml Digestionsmedium zum Gewebe hinzu.

Nach erneutem Begasen mit Carbogen für etwa 1 min und Inkubation für 15 min bei 37 °C in einem Schüttler bei 200 Zyklen/min werden die Gewebestücke durch sukzessives Aufziehen in jeweils einer 10 ml, 5 ml, 2 ml und 1 ml Glaspipette zerkleinert und durch einlagiges Filtergewebe gepresst. Die so vereinzelten Zellen werden nun fünfmal in Inkubationsmedium gewaschen (37 °C), mit Carbogen begast und jedes Mal 5 min bei 90 g zentrifugiert. Das zuletzt erhaltene Pellet wird in Inkubationsmedium resuspendiert, begast und auf Gewebekulturschalen verteilt.

### 2. Kultivierung der Zellen

Die Gewebekulturschalen mit den isolierten Zellen werden im Brutschrank bei 37°C und 5 % CO₂ kultiviert. Alle 2-3 Tage wird das Medium gewechselt. Dabei werden alle erkennbaren differenzierten Zellen entfernt.

Am siebenten Tag in Kultur werden die Zellen mit einer Lösung bestehend aus 2 ml PBS, 1 ml Trypsin und 2 ml Inkubationsmedium passagiert. Dabei lösen sich die Zellen vom Boden der Kulturschale. Die Zellsuspension wird 5 Minuten zentrifugiert, der Überstand abgesaugt und die Zellen in 2 ml Inkubationsmedium resuspendiert, auf eine mittlere Zellkulturflasche überführt und 10 ml Inkubationsmedium dazugegeben. Der Medienwechsel erfolgt alle drei Tage.

Am vierzehnten Tag in Kultur werden die Zellen erneut, aber diesmal mit 6 ml PBS, 3 ml Trypsin und 6 ml Inkubationsmedium, passagiert. Die Zellsuspension wird 5 Minuten zentrifugiert, der Überstand abgesaugt und die Zellen in 6 ml Inkubationsmedium resuspendiert, auf 3 mittlere Zellkulturflaschen überführt und jeweils 10 ml Inkubationsmedium dazugegeben.

Die Zellen werden weiter kultiviert und so oft passagiert und ausgesät, bis die Zellen einen semikonfluenten bis konfluenten Zustand erreichen. In diesem Zustand erfolgt die erfindungsgemäße Einführung und weitere Kultivierung im Wirts-Ei.

### BEISPIEL 2

Pankreas-Acini wurden von männlichen Sprague-Dawley-Ratten (20-300 g) erhalten, die narkotisiert (CO₂) und über die dorsale Aorta ausgeblutet worden waren. Eine Kanüle wurde transduodenal in den Pankreasgang eingeführt und dem Pankreas wurde von hinten 10 ml Digestionsmedium, enthaltend HEPES-Eagle-Medium (pH 7,4), 0,1 mM HEPES-Puffer (pH, 7,6), 70% (Vol./Vol.) modifiziertes Eagle-Medium, 0,5 % (Vol./Vol.) Trasylol (Bayer AG, Leverkusen, Deutschland), 1% (Gew./Vol.) Rinderserumalbumin), 2,4 mM CaCl₂ und Kollagenase (0,63 P/mg, Serva, Heidelberg, Deutschland) injiziert.

Vor der Entfernung wurde der Pankreas von anhaftendem Festgewebe, Lymphknoten und Blutgefäßen teilweise befreit. Dann wurde gesundes Pankreasgewebe in Digestionsmedium abgeholt (bei 20 °C, geringerer Stoffwechsel), das Pankreasgewebe mit einer Schere sehr fein zerkleinert, oben schwimmendes Fettgewebe abgesaugt und die Gewebesuspension mit Carbogen (Messer, Krefeld, Deutschland) begast, ohne dass die Düse in das Medium mit den Zellen gelangt (Verringerung von mechanischem Streß) und damit auf pH 7,4 eingestellt. Danach wurde die Suspension in einem 25-ml-Erlenmeyerkolben (mit Alufolie bedeckt) unter konstantem Schütteln (150-200 Zyklen pro Minute) bei 37°C in 10 ml Digestionsmedium inkubiert. Nach 15-20 Minuten wurde das oben schwimmende Fett und das Medium abgesaugt und das Gewebe wurde erneut zerkleinert und mit Medium ohne Kollagenase gespült (Vorgang mindestens zweimal wiederholen, vorzugsweise solange bis Zellfraktion transparent), worauf Digestionsmedium zugegeben und erneut etwa 1 Minute lang mit Carbogen begast wurde. Es folgte wiederum eine Digestion mit Kollagenase für 15 Minuten bei 37°C im Schüttler unter Verwendung desselben Puffers. Nach der Digestion wurden die Acini durch sukzessives Hochziehen und Ausstossen durch 10 ml-, 5 ml- und 2 ml-Glaspipetten mit engen Öffnungen dissoziiert und durch ein einlagiges Nylonsieb (Polymon PES-200/45, Angst & Pfister AG, Zürich, Schweiz) mit einer Maschengröße von etwa 250 µm filtriert. Die Acini wurden zentrifugiert (bei 37°C und 600-800 UpM in einer Beckman-GPR-Zentrifuge, entspricht etwa 50-100 g) und weiter gereinigt durch Waschen in Inkubationsmedium, enthaltend 24,5 mM HEPES (pH 7,5), 96 mM NaCl, 6 mM KCl, 1 mM MgCl₂, 2,5 mM NaH₂PO₄, 0, mM CaCl₂, 11,5 mM Glucose, 5 mM Natriumpyruvat, 5 mM Natriumglutamat, 5 mM Natriumfumarat, 1 % (Vol./Vol.) modifiziertes Eagle-Medium, 1% (Gew./Vol.) Rinderserumalbumin, mit Carbogen äquilibriert und auf pH 7,4 eingestellt. Die Waschprozedur (Zentrifugation, Absaugen, Resuspension) wurde fünfmal wiederholt. Soweit nicht anders angegeben, wird bei der obigen Isolierung bei etwa 20 °C gearbeitet.

Die Acini wurden in Inkubationsmedium resuspendiert und bei 37°C in einer angefeuchten Atmosphäre mit 5 % CO₂ kultiviert. Das acinäre Gewebe starb dabei schnell (innerhalb von zwei Tagen) ab und die sterbenden differenzierten Zellen lösten sich dabei von den benachbarten Zellen, ohne diese zu schädigen (schonende Isolierung), die nicht absterbenden Stammzellen sanken zu Boden und hefteten sich an. Die differenzierten Acinizellen sind dazu nicht in der Lage. Das Inkubationsmedium wurde am zweiten oder dritten Tag nach dem Aussäen erstmals gewechselt, wobei ein Großteil der frei schwimmenden Acini und acinären Zellen entfernt wurde. Zu diesem Zeitpunkt hatten sich die ersten Stammzellen bzw. deren Vorläufer am Boden festgesetzt und begannen sich zu teilen. Der Mediumwechsel wurde danach an jedem dritten Tag wiederholt und differenzierte acinäre Pankreaszellen wurden bei jedem Mediumwechsel entfernt.

Am siebenten Tag in Kultur wurden die Zellen mit einer Lösung bestehend aus 2 ml PBS, 1 ml Trypsin (+ 0,05 % EDTA) und 2 ml Inkubationsmedium passagiert. Dabei lösen sich die Zellen vom Boden der Kulturschale. Die Zellsuspension wurde 5 Minuten bei etwa 1000 UpM (Beckmann GPR-Zentrifuge) zentrifugiert, der Überstand abgesaugt und die Zellen in 2 ml Inkubationsmedium resuspendiert, auf eine mittlere Zellkulturflasche überführt und 10 ml Inkubationsmedium dazugegeben.

Am vierzehnten Tag in Kultur wurden die Zellen erneut, aber diesmal mit 6 ml PBS, 3 ml Trypsin/EDTA und 6 ml Inkubationsmedium, passagiert. Die Zellsuspension wird 5 Minuten bei 1000 UpM zentrifugiert, der Überstand abgesaugt und die Zellen in 6 ml Inkubationsmedium resuspendiert, auf 3 mittlere Zellkulturflaschen überführt und jeweils 10 ml Inkubationsmedium dazugegeben.

Am Tag 17 erfolgte ein drittes Passagieren auf insgesamt 6 mittlere Zellkulturflaschen und am Tag 24 ein viertes Passagieren auf insgesamt 12 mittlere Zellkulturflaschen. Spätestens jetzt waren alle primären Zellen bis auf die Stammzellen aus der Zellkultur entfernt.

Die Stammzellen können weiter kultiviert werden und so oft passagiert und ausgesät wie gewünscht. Das Aussäen erfolgt vorzugsweise jeweils in einer Dichte von 2-4 x 10⁵ Zellen/cm² in Inkubationsmedium. In diesem Zustand erfolgt wiederum die erfindungsgemäße Einführung und weitere Kultivierung im Wirts-Ei.

### BEISPIEL 3

Die Isolierung und Kultivierung aus exokrinem Gewebe der Ohrspeicheldrüse erfolgte analog dem Pankreas-Protokoll mit den folgenden Abweichungen:
1. Das exokrine Gewebe der Ohrspeicheldrüse war eine Mischung von acinärem Gewebe und tubulösem Gewebe.
2. Nachdem Speicheldrüsen weniger Proteasen und Amylasen als Pankreas enthalten, ist es möglich, das Speicheldrüsengewebe vor der Aufarbeitung einige Zeit im Kühlschrank bei etwa 4°C aufzubewahren, ohne dass das Gewebe zu sehr geschädigt wird. Im konkreten Beispielsfall betrug die Aufbewahrungszeit 15 h und brachte keine nachteiligen Folgen für die Isolierung der gewünschten Stammzellen mit sich.

### BEISPIEL 4 (Präparation von organoiden Körpern)

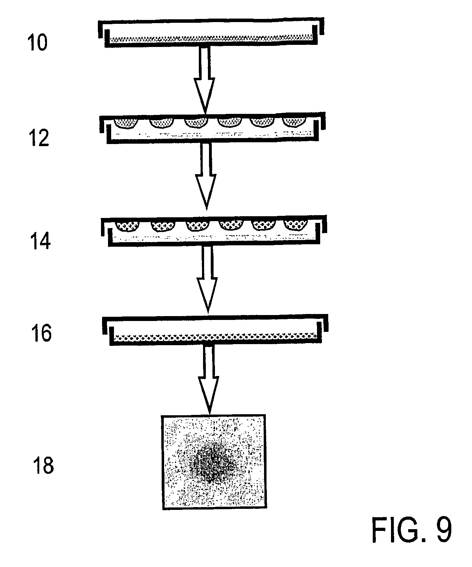

Entsprechend dem in Figur 9 dargestellten Schema wird zur Gewinnung der Stammzellen 2 acinäres Gewebe - bevorzugt der Ohrspeicheldrüse oder Bauchspeicheldrüse (Pankreas) - mechanisch und enzymatisch zerkleinert in Kultur genommen (Schritt 10 in Figur 9). Entgegen den Angaben von Bachem et al., Gastroenterol. 115:421-432 (1998), und Grosfils et al., Res. Comm. Chem. Pathol. Pharmacol. 79:99-115 (1993), werden keine Gewebeblöcke kultiviert, aus denen Zellen auswachsen sollen, sondern unter der Bedingung, dass die Zellverbände der Acini weitestgehend intakt bleiben, das Gewebe stärker zerkleinert.

Über mehrere Wochen werden diese Zellen und Zellverbände in Kulturgefäßen kultiviert. Alle 2-3 Tage wird das Medium gewechselt, wobei alle differenzierten Zellen entfernt werden. Bei den in Kultur persistierenden Zellen handelt es sich um undifferenzierte Zellen mit uneingeschränkter Teilungsfähigkeit.

In einem zweiten Schritt 12 werden ungefähr 400 bis 800 Zellen in je 20 µl Medium in hängenden Tropfen kultiviert. Dazu werden die Tropfen auf Deckel von bakteriologischen Petrischalen gegeben, umgedreht und über die mit Medium gefüllte Petrischale gelegt, sodass die Tropfen nach unten hängen.

Durch diese Art der Kultivierung bilden sich innerhalb von 48 h die als "organoid bodies" oder organoide Körper bezeichnete Zellaggregate 14, die für ungefähr 6 Tage in eine Suspensionskultur umgesetzt werden 16. Die Teilansicht 18 aus Figur 9 zeigt eine mikroskopische Aufnahme eines solchen organoiden Körpers.

Die in Suspensionskultur wachsenden organoiden Körper bilden neue organoide Körper, die auch bei Einzelzellen die Bildung von neuen organoide Körper induzieren. Die Zellen sind sowohl als organoide Körper als auch als Einzelzellen einfrierbar und behalten dabei ihre Vitalität und ihr Differenzierungspotenzial.

Die folgenden Beispiele 5 und 6 beschreiben detailliert zwei Arbeitsprotokolle zur Herstellung von organoiden Körpern und differenzierten Zellen.

### BEISPIEL 5

Die undifferenzierten Zellen werden mit einer Lösung aus 10 ml PBS, 4 ml Trypsin, 8 ml Differenzierungsmedium abtrypsiniert und 5 Minuten abzentrifugiert. Das resultierende Pellet wird so in Differenzierungsmedium resuspendiert, dass sich eine Verdünnung von 3000 Zellen je 100 µl Medium einstellt. Anschließend werden die Zellen nochmals gut mit einer 3 ml Pipette suspendiert.

Von bakteriologischen Petrischalen, die vorher mit jeweils 15 ml PBS (37 °C) pro Platte beschichtet worden sind, wird der Deckel abgenommen und umgedreht. Mit Hilfe einer automatischen Pipette werden auf einen Deckel ca. fünfzig 20 µl Tropfen gegeben. Der Deckel wird dann schnell umgedreht und auf die mit Differenzierungsmedium gefüllte Petrischale gegeben, sodass die Tropfen nach unten hängen. Die Petrischalen werden anschließend vorsichtig in den Brutschrank gestellt und für 48 h inkubiert.

Daraufhin werden die in den hängenden Tropfen aggregierten Zellen (organoide Körper) aus jeweils vier Deckeln in je eine bakteriologische Petrischale mit 5 ml Inkubationsmedium mit 20 % FKS überführt und für weitere 96 h kultiviert.

Die organoiden Körper werden nun vorsichtig mit einer Pipette aufgesammelt, und in mit 0,1 % Gelatine beschichtete Zellkulturgefäße mit Differenzierungsmedium überführt. In einer besonders bevorzugten Ausgestaltung des Verfahrens verwendet man als Kulturgefäß mit 0,1 % Gelatine beschichtete 6 cm Petrischalen, in die 4 ml Differenzierungsmedium vorgelegt wurde und die anschließend mit je 6 organoiden Körper beschickt werden. Ein weiteres bevorzugtes Kulturgefäß sind mit 0,1 % Gelatine beschichtete Chamber Slides, in die 3 ml Differenzierungsmedium vorgelegt wurde und die anschließend mit je 3-8 organoiden Körper beschickt werden. Daneben können auch. 24-Mulden-Mikrotiterplatten verwendet werden, die mit 0,1 % Gelatine beschichtet wurden und in die je 1,5 ml pro Mulde Differenzierungsmedium vorgelegt wurde und anschließend mit je 4 organoiden Körper beschickt werden.

Derart kultiviert, ist die Differenzierungsfähigkeit der Zellen in den organoiden Körper aktiviert und die Zellen differenzieren sich in Zellen der drei Keimblätter Mesoderm, Entoderm und Ektoderm. Die Zellen können sowohl als organoide Körper als auch als einzelne Zellen gelagert und kultiviert werden und behalten ihre Pluripotenz.

Die Differenzierung kann als Vordifferenzierung außerhalb des Wirts-Ei erfolgen oder durch den Zusatz der genannten Differenzierungsfaktoren bei der Kultivierung im Wirts-Ei aktiviert werden. Es wurden bisher u.a. glatte Muskelzellen, Neuronen, Gliazellen, Epithelzellen, Fettzellen, Herzzellen, Nierenzellen, Fibroblasten (z.B. Haut- und Sehnenfibroblasten), Chondrozyten, endokrine und exokrine Drüsenzellen und damit Zelltypen aller drei Keimblätter nachgewiesen.

### BEISPIEL 6

Für die Kultivierung im Wirts-Ei wurden vorzugsweise Stammzellen nach dem 42. Tag der Kultivierung verwendet. Die Verwendung von Stammzellen nach der 3. oder 4. Passage oder von Zellen, die bei der Temperatur von flüssigem Stickstoff 12-18 Monate lang gelagert worden waren, war ebenfalls problemlos möglich.

Zunächst wurden die Zellen in Differenzierungsmedium mit der oben angegebenen Zusammensetzung überführt und auf eine Dichte von etwa 3 x 10⁴ Zellen/ml eingestellt; z.B. durch Trypsinbehandlung einer Stammzellkultur in Nährmedium, 5-minütige Zentrifugation bei 1000 UpM und Resuspendierung des Pellets in Differenzierungsmedium und Verdünnung soweit erforderlich.

Anschließend wurden mit einer 20-µl-Pipette ca. 50 20-µl-Tropfen (600 Zellen/20 µl) auf die Innenseite des Deckels einer bakteriologischen Petrischale gegeben (gestopfte Spitzen) und die Deckel vorsichtig auf die mit PBS gefüllten Petrischalen gestülpt, sodass die Tropfen nach unten hängen. Für jeden Deckel wurde eine neue Spitze verwendet. Die Petrischalen wurden anschließend vorsichtig in den Brutschrank gestellt und 48 h lang bei 37°C inkubiert.

Danach wurden die in den hängenden Tropfen aggregierten Zellen, die organoiden Körper, aus jeweils vier Deckeln in je eine bakteriologische Petrischale mit 5 ml Inkubationsmedium mit 20% FKS überführt (Deckel schräg halten und die OBs mit etwa 2,5 ml Nährmedium abspülen) und für weitere 5-9 Tage, vorzugsweise 96 h, kultiviert.

Die organoiden Körper wurden nun vorsichtig mit einer Pipette aufgesammelt und in mit 0,1 % Gelatine beschichtete Zellkulturgefäße mit Differenzierungsmedium überführt. Die OB vermehrten sich nun und wuchsen in zum Teil einzelnen Zellkolonien, die wieder vermehrt, vereinzelt und vermehrt werden konnten. In einer besonders bevorzugten Ausgestaltung des Verfahrens wurden als Kulturgefäß mit 0,1 % Gelatine beschichtete 6 cm Petrischalen verwendet, in die 4 ml Differenzierungsmedium vorgelegt worden war, und diese mit je 6 organoide Körper beschickt. Ein weiteres bevorzugtes Kulturgefäß waren mit 0,1 % Gelatine beschichtete Chamber Slides, in die 3 ml Differenzierungsmedium vorgelegt wurde und die anschließend mit je 3-8 organoiden Körper beschickt wurden, und Thermanox-Platten (Nalge Nonc International, USA) für elektronenmikroskopische Studien. Ein weitere Alternative waren 24-Mulden-Mikrotiterplatten, die mit 0,1 % Gelatine beschichtet wurden und in die je 1,5 ml pro Mulde Differenzierungsmedium vorgelegt wurde und die anschließend mit je 4 organoiden Körper beschickt wurden.

Anschließend wurden die organoiden Körper oder Gewebekörper im Differenzierungsmedium suspendiert. Im suspendierten Zustand erfolgt die Übertragung in ein Hühnerei. Hierzu wurde eine ausgezogene, hydrophobierte Glaskapillare verwendet. Dabei wurde ein Suspensionsvolumen eingestellt, das rd. 2- bis 20-fach größer als das Zellmaterialvolumen ist. Die weitere Kultivierung im Hühnerei erfolgte mit einer Begasung mit einem Luft-CO₂-Gemisch (CO₂-Gehalt: 5%).

### BEISPIEL 7

Zur Isolierung und Kultivierung von humanen adulten Stammzellen wurde humanes Gewebe von erwachsenen Patienten unmittelbar nach einem chirurgischen Eingriff erhalten und sofort aufgearbeitet. Aus dem chirurgisch entfernten Gewebe, z.B. Pankreas- oder Speicheldrüsengewebe, wurde gesundes Gewebe abgetrennt und in Digestionsmedium aufgenommen. Dieses Gewebe wurde dann analog dem für die Ratte beschriebenen Protokoll aufgearbeitet und die Stammzellen in analoger Weise isoliert und kultiviert.

### BEISPIEL 8

Bei der Einführung von Zellmaterial, das von Fischen einer ersten Art stammt, in Fischeier einer anderen Art, wird beispielsweise von Stammzellen ausgegangen, die aus der Pankreas von Aalen gewonnen wurden. Diese Stammzellen werden nach den oben beschriebenen Techniken in Eier von Forellen oder Karpfen implantiert und dort kultiviert. Im Ergebnis können Aale ausgebrütet werden, was im Rahmen von Zuchtverfahren bisher nicht möglich war, da Aale nur in der Tiefsee laichen.

Die in der vorstehenden Beschreibung, den Ansprüchen und den Zeichnungen offenbarten Merkmale der Erfindung können sowohl einzeln oder auch in Kombination für die Verwirklichung der Erfindung in ihren verschiedenen Ausgestaltungen von Bedeutung sein.

## Patentansprüche

1. Verfahren zum Kultivieren von Stammzellen (2, 3, 5), ausgenommen humane embryonale Stammzellen, umfassend die Schritte:
- Einführung von Zellmaterial, das mindestens eine Stammzelle (2, 3, 5) eines Donor-Organismus umfasst, in ein Wirts-Ei (4) mit einem Nährstoffreservoir, wobei das Zellmaterial im Wirts-Ei (4) in einer Kultivierungsblase (46) angeordnet wird, die eine Kultivierungssubstanz enthält, und
- Kultivierung des Zellmaterials im Wirts-Ei (4), wobei die Kultivierung ein differenziertes Wachstum des Zellmaterials umfasst und wobei das differenzierte Wachstum des Zellmaterials durch stoffliche, mechanische oder thermische Wachstumsbedingungen beeinflusst wird.

2. Verfahren nach Anspruch 1, bei dem das Zellmaterial in ein Wirts-Ei (4) eingeführt wird, das nicht aus einem Säugerorganismus stammt.

3. Verfahren nach Anspruch 2, bei dem das Zellmaterial in ein Wirts-Ei (4) eines Organismus eingeführt wird, der aus der Gruppe ausgewählt ist, die die Klassen Vögel, Fische, Amphibien, Reptilien und Insekten umfasst.

4. Verfahren nach mindestens einem der vorhergehenden Ansprüche, bei dem das Zellmaterial in ein unbefruchtetes Wirts-Ei (4) eingeführt wird.

5. Verfahren nach mindestens einem der vorhergehenden Ansprüche 1 bis 3, bei dem das Zellmaterial in ein befruchtetes Wirts-Ei (4) eingeführt wird.

6. Verfahren nach Anspruch 5, bei dem vor der Einführung des Zellmaterials eine Keimscheibe aus dem Wirts-Ei (4) entfernt oder deaktiviert wird.

7. Verfahren nach mindestens einem der vorhergehenden Ansprüche, bei dem die mindestens eine Stammzelle (2) eine adulte Stammzelle ist.

8. Verfahren nach Anspruch 7, bei dem die adulte Stammzelle aus einer exokrinen Drüse (1) des Donor-Organismus stammt.

9. Verfahren nach mindestens einem der Ansprüche 1 bis 6, bei dem die mindestens eine Stammzelle (2) eine nicht-humane, embryonale Stammzelle des Donor-Organismus ist.

10. Verfahren nach mindestens einem der vorhergehenden Ansprüche, bei dem das in das Wirts-Ei (4) eingeführte Zellmaterial mindestens ein Aggregat (3, 5) von Stammzellen umfasst, das einen Zellkörper bildet.

11. Verfahren nach nach mindestens einem der vorhergehenden Ansprüche, bei dem eine Injektion einer Kultivierungssubstanz mit mindestens einem Differenzierungsfaktor und/oder mindestens einem Wachstumsfaktor in das Wirts-Ei (4) vorgesehen ist.

12. Verfahren nach mindestens einem der vorhergehenden Ansprüche, bei dem während der Kultivierung eine Messung mindestens einer Eigenschaft des Zellmaterials vorgesehen ist.

13. Verfahren nach Anspruch 12, bei dem die gemessene Eigenschaft für einen Vitalitätszustand des Zellmaterials charakteristisch ist.

14. Verfahren nach mindestens einem der vorhergehenden Ansprüche, bei dem die Kultivierung des Zellmaterials in das Wirts-Ei (4) beendet wird, wenn ein vorbestimmter Kultivierungszustand erreicht ist.

15. Verfahren nach mindestens einem der vorhergehenden Ansprüche, bei dem der Kultivierungszustand eine Kultivierungsdauer, eine Größe und/oder einen Differenzierungsgrad des kultivierten Zellmaterials umfasst.

16. Verfahren nach mindestens einem der vorhergehenden Ansprüche, bei dem das Zellmaterial im Wirts-Ei (4) an einer Position angeordnet wird, die im Wirts-Ei (4) der Position der Keimscheibe entspricht.

17. Verfahren nach mindestens einem der vorhergehenden Ansprüche, bei dem die Kultivierungssubstanz in der Kultivierungsblase (46) während der Kultivierung des Zellmaterials im Wirts-Ei (4) kontinuierlich oder nach vorbestimmten Zeitabständen wiederholt ausgetauscht wird.

18. Verfahren nach mindestens einem der vorhergehenden Ansprüche, bei dem das Zellmaterial in ein Wirts-Ei (4) eingeführt wird, dessen natürliches Nährstoffreservoir zumindest teilweise durch eine Kultivierungssubstanz ausgetauscht ist.

19. Verfahren nach Anspruch 18, bei dem der Austausch während der Kultivierung des Zellmaterials im Wirts-Ei (4) erfolgt.

20. Verfahren nach Anspruch 18 oder 19, bei dem der Austausch durch Einbringen von Tropfen der Kultivierungssubstanz in das Nährstoffreservoir des Wirts-Ei (4) erfolgt.

21. Verfahren nach Anspruch 20, bei dem die Tropfen in einem membranumhüllten Zustand in das Wirts-Ei (4) eingeführt werden.

22. Verfahren nach einem der Ansprüche 18 bis 21, bei dem Eiweiß (45) und/oder Eidotter (43) des Wirts-Ei (4) komplett durch die Kultivierungssubstanz ersetzt wird.

23. Verfahren nach mindestens einem der vorhergehenden Ansprüche, bei dem während der Kultivierung des Zellmaterials im Wirts-Ei (4) ein Stoff- und/oder Temperaturgradient erzeugt wird.

24. Verfahren nach mindestens einem der vorhergehenden Ansprüche, bei dem mit mindestens einem Feststoff geometrische Randbedingungen für die Kultivierung des Zellmaterials vorgegeben werden.

25. Verfahren nach Anspruch 24, bei dem der Feststoff durch einen Trägerpartikel gebildet wird, der während der Kultivierung ein Substrat für die Stammzellen bildet.

26. Verfahren nach Anspruch 25, bei dem das Zellmaterial im Verbund mit dem Trägerpartikel in das Wirts-Ei (4) eingeführt wird.

27. Verfahren nach Anspruch 24, bei dem der Feststoff durch eine im Wirts-Ei (4) ausgehärtete Substanz gebildet wird.

28. Zellkultur, die umfasst:
- ein Wirts-Ei (4) mit einem Nährstoffreservoir, und
- Zellmaterial, das mindestens eine Stammzelle, ausgenommen eine humane embryonale Stammzelle, eines Donor-Organismus umfasst und im Wirts-Ei (4) angeordnet ist,
wobei das Wirts-Ei (4) eine Kultivierungsblase (46) mit einer Kultivierungssubstanz enthält, in der das Zellmaterial angeordnet ist.

29. Zellkultur nach Anspruch 28, bei der das Zellmaterial nicht aus einem Säugerorganismus stammt.

30. Zellkultur nach Anspruch 29, bei der das Zellmaterial aus einem Organismus stammt, der aus der Gruppe ausgewählt ist, die die Klassen Vögel, Fische, Reptilien und Insekten umfasst.

31. Zellkultur nach einem der Ansprüche 28 bis 30, bei der das Wirts-Ei (4) ein unbefruchtetes Wirts-Ei (4) ist.

32. Zellkultur nach einem der Ansprüche 28 bis 31, bei der das Wirts-Ei (4) eine deaktivierte Keimscheibe oder keine Keimscheibe enthält.

33. Zellkultur nach einem der Ansprüche 28 bis 32, bei der die mindestens eine Stammzelle (2) eine adulte Stammzelle ist.

34. Zellkultur nach einem der Ansprüche 28 bis 32, bei der die mindestens eine Stammzelle eine nicht-humane embryonale Stammzellen des Donor-Organismus ist.

35. Zellkultur nach einem der Ansprüche 28 bis 34, bei der das Zellmaterial mindestens ein Aggregat (3, 5) von Stammzellen umfasst, das einen Zellkörper bildet.

36. Zellkultur nach einem der Ansprüche 28 bis 35, bei der im Wirts-Ei (4) eine Kultivierungssubstanz mit mindestens einem Differenzierungsfaktor und/oder mindestens einem Wachstumsfaktor angeordnet ist.

37. Zellkultur nach einem der Ansprüche 28 bis 36, bei der mindestens ein Teil des Nährstoffreservoirs durch eine von außen in das Wirts-Ei (4) eingeführte Kultivierungssubstanz gebildet wird.

38. Zellkultur nach einem der Ansprüche 28 bis 37, bei der das Wirts-Ei (4) mindestens einen Feststoff zur Vorgabe geometrischer Randbedingungen für die Kultivierung des Zellmaterials enthält.

39. Zellkultur nach Anspruch 38, bei der der mindestens eine Feststoff mindestens einen Trägerpartikel und/oder mindestens eine im Wirts-Ei (4) ausgehärtete Substanz umfasst.

40. Verwendung von einem Ei eines Wirts-Organismus zur Kultivierung von Stammzellen, die nicht vom Wirts-Organismus strammen, sondern von einem Donor-Organismus, bei dem es sich nicht um einen Säuger handelt.

41. Verwendung von einem Ei als Inkubatoreinrichtung für von außen in das Ei eingesetzte Stammzellen von einem Donor-Organismus, bei dem es sich nicht um einen Säuger handelt.

42. Verwendung nach Anspruch 40 oder 41, bei der Organismus aus der Gruppe ausgewählt ist, die die Klassen Vögel, Fische, Reptilien und Insekten umfasst.

## Claims

1. A method for cultivating stem cells (2, 3, 5), except human embryonic stem cells, comprising the steps:
- introduction of cell material comprising at least one stem cell (2, 3, 5) of a donor organism into a host egg (4) with a nutrient reservoir, wherein the cell material in the host egg (4) is arranged in a cultivation bubble (46) containing a cultivation substance, and
- cultivation of the cell material in the host egg (4), wherein the cultivation comprises a differentiated growth of the cell material and wherein the differentiated growth of the cell material is influenced by material, mechanical or thermal growth conditions.

2. The method according to Claim 1, in which the cell material is introduced into a host egg (4) that does not stem from a mammalian organism.

3. The method according to Claim 2, in which the cell material is introduced into a host egg (4) of an organism selected from the group comprising the classes birds, fish, amphibians, reptiles and insects.

4. The method according to at least one of the preceding claims, in which the cell material is introduced into an unfertilized host egg (4).

5. The method according to at least one of the preceding claims 1 to 3, in which the cell material is introduced into a fertilized host egg (4).

6. The method according to Claim 5, in which a germ disk is removed from the host egg (4) or deactivated prior to the introduction of the cell material.

7. The method according to at least one of the preceding claims, in which the at least one stem cell (2) is an adult stem cell.

8. The method according to Claim 7, in which the adult stem cell stems from an exocrine gland (1) of the donor organism.

9. The method according to at least one of the claims 1 to 6, in which the at least one stem cell (2) is a non-human embryonic stem cell of the donor organism.

10. The method according to at least one of the preceding claims, in which the cell material introduced into the host egg (4) comprises at least one aggregate (3, 5) of stem cells that forms a cell body.

11. The method according to at least one of the preceding claims, in which an injection of a cultivation substance with at least one differentiation factor and/or at least one growth factor into the host egg (4) is provided.

12. The method according to at least one of the preceding claims, in which a measuring of at least one property of the cell material is provided during the cultivation.

13. The method according to Claim 12, in which the property measured is characteristic for a vitality state of the cell material.

14. The method according to at least one of the preceding claims, in which the cultivation of the cell material in the host egg (4) is terminated when a predetermined cultivation state has been reached.

15. The method according to at least one of the preceding claims, in which the cultivation state comprises a cultivation duration, a size and/or a degree of differentiation of the cultivated cell material.

16. The method according to at least one of the preceding claims, in which the cell material in the host egg (4) is arranged at a position that corresponds in the host egg (4) to the position of the germ disk.

17. The method according to according to at least one of the preceding claims, in which the cultivation substance in the cultivation bubble (46) is continuously exchanged during the cultivation of the cell material in the host egg (4) or is repeatedly exchanged in accordance with predetermined time intervals.

18. The method according to at least one of the preceding claims, in which the cell material is introduced into a host egg (4) whose natural nutrient reservoir is exchanged at least partially by a cultivation substance.

19. The method according to Claim 18, in which the exchange takes place during the cultivation of the cell material in the host egg (4).

20. The method according to Claim 18 or 19, in which the exchange takes place by introducing drops of the cultivation substance into the nutrient reservoir of the host egg (4).

21. The method according to Claim 20, in which the drops are introduced in a membrane-surrounded state into the host egg (4).

22. The method according to one of Claims 18 to 21, in which egg white (45) and/or egg yolk (43) of the host egg (4) is/are completely replaced by the cultivation substance.

23. The method according to at least one of the preceding claims, in which a material gradient and/or temperature gradient is/are produced during the cultivation of the cell material in the host egg (4).

24. The method according to at least one of the preceding claims, in which geometric boundary conditions for the cultivation of the differentiation medium are given with at least one solid.

25. The method according to Claim 24, in which the solid is formed by a carrier particle that forms a substrate for the stem cells during the cultivation.

26. The method according to Claim 25, in which the cell material is introduced in a compound with the carrier particle into the host egg (4).

27. The method according to Claim 26, in which the solid is formed by a substance hardened in the host egg (4).

28. A cell culture that comprises:
- a host egg (4) with a nutrient reservoir, and
- cell material that comprises at least one stem cell, except a human embryonic stem cell, of a donor organism and is arranged in the host egg (4), wherein the host egg (4) contains a cultivation bubble (46) with a cultivation substance in which the cell material is arranged.

29. The cell culture according to Claim 28, in which the cell material does not stem from a mammalian organism.

30. The cell culture according to Claim 29, in which the cell material stems from an organism selected from the group comprising the classes birds, fish, amphibians, reptiles and insects.

31. The cell culture according any one of Claims 28 to 30, in which the host egg (4) is an unfertilized host egg (4).

32. The cell culture according any one of Claims 28 to 31, in which the host egg (4) contains a deactivated germ disk or no germ disk.

33. The cell culture according any one of Claims 28 to 32, in which the at least one stem cell (2) is an adult stem cell.

34. The cell culture according any one of Claims 28 to 32, in which the at least one stem cell is a non-human embryonic stem cell of the donor organism.

35. The cell culture according any one of Claims 28 to 34, in which the cell material comprises at least one aggregate (3, 5) of stem cells that forms a cell body.

36. The cell culture according any one of Claims 28 to 35, in which a cultivation substance with at least one differentiation factor and/or at least one growth factor is arranged in the host egg (4).

37. The cell culture according any one of Claims 28 to 36, in which at least a part of the nutrient reservoir is formed by a cultivation substance introduced from the outside into the host egg (4).

38. The cell culture according any one of Claims 28 to 37, in which the host egg (4) contains at least one solid for giving geometric boundary conditions for the cultivation of the cell material.

39. The cell culture according to Claim 38, in which the at least one solid comprises at least one carrier particle and/or at least one substance hardened in the host egg (4).

40. The use of an egg of a host organism for the cultivation of stem cells, that do not stem from the host organism but from a donor organism which is not a mammalian organism.

41. The use of an egg as incubator device for stem cells, inserted from the outside into the egg, wherein the stem cells stem from a donor organism which is not a mammalian organism.

42. The use according to claim 40 or 41, in which the organism is selected from the group comprising the classes birds, fish, amphibians, reptiles and insects.

## Revendications

1. Procédé de culture de cellules souches (2, 3, 5), à l'exception des cellules souches embryonnaires humaines, comprenant les étapes consistant à :
- introduire le matériel cellulaire qui comprend au moins une cellule souche (2, 3, 5) d'un organisme donneur, dans un ovule hôte (4) avec un réservoir de nutriments, où le matériel cellulaire dans l'ovule hôte (4) est disposé dans une vésicule de culture (46) qui contient une substance de culture, et
- cultiver le matériel cellulaire dans l'ovule hôte (4), où la culture comprend une croissance différenciée du matériel cellulaire et où la croissance différenciée du matériel cellulaire est influencée par les conditions de croissance substantielles, mécaniques ou thermiques.

2. Procédé selon la revendication 1, dans lequel le matériel cellulaire est introduit dans une cellule hôte (4) qui ne provient pas d'un organisme mammifère.

3. Procédé selon la revendication 2, dans lequel le matériel cellulaire est introduit dans un ovule hôte (4) d'un organisme, qui est choisi dans le groupe qui comprend les classes oiseaux, poissons, amphibies, reptiles et insectes.

4. Procédé selon au moins une des revendications précédentes, dans lequel le matériel cellulaire est introduit dans un ovule hôte (4) non fécondé.

5. Procédé selon au moins une des revendications précédentes 1 à 3, dans lequel le matériel cellulaire est introduit dans un ovule hôte (4) fécondé.

6. Procédé selon la revendication 5, dans lequel, avant l'introduction du matériel cellulaire, une discoblastule est retirée ou désactivée de l'ovule hôte (4).

7. Procédé selon au moins une des revendications précédentes, dans lequel la au moins une cellule souche (2) est une cellule souche adulte.

8. Procédé selon la revendication 7, dans lequel la cellule souche adulte provient d'une glande exocrine (1) de l'organisme donneur.

9. Procédé selon au moins une des revendications 1 à 6, dans lequel la au moins une cellule souche (2) est une cellule souche embryonnaire non humaine de l'organisme donneur.

10. Procédé selon au moins une des revendications précédentes, dans lequel le matériel cellulaire introduit dans l'ovule hôte (4) comprend au moins un agrégat (3, 5) de cellules souches, qui forme un corps cellulaire.

11. Procédé selon au moins une des revendications précédentes, dans lequel une injection d'une substance de culture ayant au moins un facteur de différenciation et/ou au moins un facteur de croissance dans l'ovule hôte (4) est prévue.

12. Procédé selon au moins une des revendications précédentes, dans lequel une mesure d'au moins une propriété du matériel cellulaire est prévue pendant la culture.

13. Procédé selon la revendication 12, dans lequel la propriété mesurée est caractéristique d'un état de vitalité du matériel cellulaire.

14. Procédé selon au moins une des revendications précédentes, dans lequel la culture du matériel cellulaire dans l'ovule hôte (4) se termine quand un état de culture prédéterminé est atteint.

15. Procédé selon au moins une des revendications précédentes, dans lequel l'état de culture comprend une durée de culture, une taille et/ou un degré de différenciation du matériel cellulaire cultivé.

16. Procédé selon au moins une des revendications précédentes, dans lequel le matériel cellulaire dans l'ovule hôte (4) est disposé en une position, qui correspond à la position de la discoblastule dans l'ovule hôte (4).

17. Procédé selon au moins une des revendications précédentes, dans lequel la substance de culture est remplacée de manière répétée en continu ou à intervalles prédéterminés dans la vésicule de culture (46) pendant la culture du matériel cellulaire dans l'ovule hôte (4).

18. Procédé selon au moins une des revendications précédentes, dans lequel le matériel cellulaire est introduit dans un ovule hôte (4), dont le réservoir de nutriment naturel est au moins partiellement remplacé par une substance de culture.

19. Procédé selon la revendication 18, dans lequel le remplacement s'effectue pendant la culture du matériel cellulaire dans l'ovule hôte (4).

20. Procédé selon la revendication 18 ou 19, dans lequel le remplacement s'effectue par introduction de gouttes de la substance de culture dans le réservoir de nutriment de l'ovule hôte (4).

21. Procédé selon la revendication 20, dans lequel les gouttes sont introduites dans l'ovule hôte (4) à l'état enveloppé dans la membrane.

22. Procédé selon l'une des revendications 18 à 21, dans lequel le blanc (45) et/ou le jaune (43) de l'ovule hôte (4) est complètement remplacé par la substance de culture.

23. Procédé selon au moins une des revendications précédentes, dans lequel un gradient de substance et/ou de température est produit pendant la culture du matériel cellulaire dans l'ovule hôte (4).

24. Procédé selon au moins une des revendications précédentes, dans lequel des conditions cadres géométriques avec au moins une matière solide pour la culture du matériel cellulaire sont prédéterminées.

25. Procédé selon la revendication 24, dans lequel la matière solide est formée par une particule support, qui forme un substrat pour les cellules souches pendant la culture.

26. Procédé selon la revendication 25, dans lequel le matériel cellulaire est introduit dans l'ovule hôte (4) en association avec la particule support.

27. Procédé selon la revendication 24, dans lequel la matière solide est formée par une substance durcie dans l'ovule hôte (4).

28. Culture cellulaire, comprenant :
- un ovule hôte (4) avec un réservoir de nutriment, et
- un matériel cellulaire, qui comprend au moins une cellule souche, à l'exception d'une cellule souche embryonnaire humaine, d'un organisme donneur et est disposé dans l'ovule hôte (4),
l'ovule hôte (4) contenant une vésicule de culture (46) avec une substance de culture, dans laquelle est disposé le matériel cellulaire.

29. Culture cellulaire selon la revendication 28, dans laquelle le matériel cellulaire ne provient pas d'un organisme de mammifère.

30. Culture cellulaire selon la revendication 29, dans lequel le matériel cellulaire provient d'un organisme qui est choisi dans le groupe comprenant les classes oiseaux, poissons, reptiles et insectes.

31. Culture cellulaire selon l'une des revendications 28 à 30, dans laquelle l'ovule hôte (4) est un ovule hôte (4) non fécondé.

32. Culture cellulaire selon l'une des revendications 28 à 31, dans laquelle l'ovule hôte (4) comprend une discoblastule désactivée ou aucune discoblastule.

33. Culture cellulaire selon l'une des revendications 28 à 32, dans laquelle la au moins une cellule souche (2) est une cellule souche adulte.

34. Culture cellulaire selon l'une des revendications 28 à 32, dans laquelle la au moins une cellule souche est une cellule souche embryonnaire non humaine de l'organisme donneur.

35. Culture cellulaire selon l'une des revendications 28 à 34, dans laquelle le matériel cellulaire comprend au moins un agrégat (3, 5) de cellules souches, qui forme un corps cellulaire.

36. Culture cellulaire selon l'une des revendications 28 à 35, dans laquelle une substance de culture ayant au moins un facteur de différenciation et/ou au moins un facteur de croissance est disposée dans l'ovule hôte (4).

37. Culture cellulaire selon l'une des revendications 28 à 36, dans laquelle au moins une partie du réservoir de nutriment est formée par une substance de culture introduite de l'extérieure dans l'ovule hôte (4).

38. Culture cellulaire selon l'une des revendications 28 à 37, dans laquelle l'ovule hôte (4) comprend au moins une matière solide pour la prédétermination des conditions cadres géométriques pour la culture du matériel cellulaire.

39. Culture cellulaire selon la revendication 38, dans laquelle la au moins une matière solide comprend au moins une particule support et/ou au moins une substance durcie dans l'ovule hôte (4).

40. Utilisation d'un ovule d'un organisme hôte pour la culture de cellules souches qui ne proviennent pas d'un organisme hôte, mais d'un organisme donneur, qui n'est pas un mammifère.

41. Utilisation d'un ovule comme système incubateur pour des cellules souches, insérées par l'extérieur dans l'ovule, d'un organisme donneur, qui n'est pas un mammifère.

42. Utilisation selon la revendication 40 ou 41, dans laquelle l'organisme est choisi dans le groupe qui comprend les classes oiseaux, poissons, reptiles et insectes.
